# EUROPEAN PATENT APPLICATION

(11) **EP 3 862 422 A1**
(43) Date of publication of application: **11.08.2021**
(21) Application number: 20216308.5
(22) Date of filing: 23.06.2014
(51) Int. Cl.: C12N 5/00

(54) **EXTRACELLULAR MATRIX COMPOSITION BEADS FOR CELL CULTURE**

(30) Priority: 24.06.2013 US 201361838693 P
(62) Divisional of application: 14816591.3
(71) Applicant: Celularity Inc., Florham Park, NJ 07932 (US)
(72) Inventor: HARIRI, Robert J., Florham Park, NJ 07932 (US); BHATIA, Mohit, Manalapan, NJ 07726 (US)
(74) Representative: Mewburn Ellis LLP

(57) **Abstract**

Provided herein are methods of culturing cells using microcarriers that include extracellular matrix (ECM).

## Description

This application claims priority to U.S. Provisional Patent Application No. 61/838,693, filed June 24, 2013, the disclosure of which is incorporated herein by reference in its entirety.

### 1. FIELD

Provided herein are beads comprising extracellular matrix, methods of producing the beads, and methods of cell culture using the beads.

### 2. BACKGROUND

Extracellular matrix (ECM) comprises protein that forms many structures in the body including tendons, ligaments, and sheets that support skin and internal organs. There is a need in the art for improved methods of culturing cells using microcarriers, e.g., for bioreactor culture. Provided herein are such improved microcarriers and culture methods.

### 3. SUMMARY

In one aspect, provided herein is a method of culturing cells, comprising contacting said cells with a plurality of microcarriers under conditions in which said cells can proliferate, wherein said microcarriers comprise extracellular matrix (ECM), and wherein said cells are adherent to said ECM. In certain embodiments, the microcarriers consist essentially of, or consist of, ECM. In certain other embodiments, the microcarriers comprise a second composition in addition to said ECM, for example, one or more of glass, dextran, DEAE-dextran, polystyrene, acrylamide, or collagen. In a specific embodiment, the ECM forms an exterior of said microcarrier, and said second composition forms an interior of said microcarrier. The ECM may cover a portion of, substantially the entire surface of, or the entire surface of, said microcarrier. In other words, the microcarrier may comprise a composition partly or completely covered by, or coated with, the ECM. In any of the embodiments herein, the microcarrier is substantially in the shape of a sphere. In various specific embodiments, the microcarriers average, e.g., between 10 micrometers to 1000 micrometers in diameter; between 50 and 200 micrometers in diameter, between 100 and 250 micrometers in diameter; between 150 and 300 micrometers in diameter; between 200 and 350 micrometers in diameter; or between 250 and 400 micrometers in diameter.

In any of the embodiments herein, said culturing is performed in a bioreactor, e.g., a batch reactor, a fed batch reactor, or a continuous stirred-tank reactor.

In certain embodiments, said ECM is base-treated, detergent-treated ECM. In certain other embodiments, said ECM is detergent-treated ECM that has not been contacted with a base. In one embodiment, the ECM comprises collagen, and comprises between 3% and 5% elastin by dry weight, and less than 0.01% laminin or less than 0.01% fibronectin by dry weight. In another embodiment, said ECM comprises collagen, and comprises between 3% and 5% elastin by dry weight, less than 0.01% laminin and less than 0.01% fibronectin by dry weight. In another specific embodiment, said ECM comprises at least 80% collagen by dry weight and at least 10% elastin by dry weight.

The ECM used in the methods described herein may be defined by the process of its making. For example, in any of the embodiments herein, ECM may be prepared in part, e.g., by a method comprising the steps, in order, of: (a) macerating placental tissue; (b) suspending the tissue in an osmotic shock solution; (c) suspending the tissue in a solution that comprises a detergent; (d) suspending the tissue in a basic solution; (e) rinsing said tissue with water; and (f) drying said tissue to form said ECM. In any of the embodiments herein, the ECM may be prepared in part by a method comprising the steps, in order, of: (a) macerating placental tissue; (b) suspending the tissue in an osmotic shock solution; (c) suspending the tissue in a solution that comprises detergent; (e) rinsing said tissue with water; and (f) drying said tissue to form said ECM. In more specific embodiments, the drying steps above may comprise the steps, in order, of: (a) lyophilizing the ECM; (b) rehydrating the ECM; and (c) drying the ECM at a temperature of 40°C to 70°C, or 50°C to 70°C, or 50°C to 80°C to produce said ECM composition. ECM produced according to this drying method is referred to herein as "double-dried ECM." The detergent may be any detergent, e.g., a mild detergent, e.g., deoxycholic acid. The base, when used to make the ECM, may be any base, e.g., a strong base, e.g., ammonium hydroxide, potassium hydroxide, or sodium hydroxide. The base may be used, e.g., at a concentration of less than 0.5M, 0.4M, 0.3M, 0.2M, 0.1M or 0.5M.

In certain embodiments, the ECM used in the methods described herein has not been modified with a chemical (other than a base or detergent) or protease.

In any of the embodiments herein, the ECM may be non-human ECM (that is, ECM from a non-human source). The ECM may be human ECM, e.g., ECM from human placental tissue.

Any type of cell may be cultured using the methods provided herein, which include use of microcarriers comprising ECM, as long as the cells can attach to the ECM. In certain specific embodiments, the cells are insect cells. In certain other embodiments, the cells are mammalian cells, e.g., adult (terminally differentiated) cells or stem cells. In a specific embodiment, the cells are fibroblasts. In other specific embodiments, the cells are, e.g., keratinocytes, vascular endothelial cells, hepatocytes, stellate cells, vascular smooth muscle cells, hepatocyte progenitors, primary chick embryo neuronal cells, adherent CHO or BHK cells, macrophages, 293 cells, L929 cells, immortalized mouse testicular germ cells, 3T3 cells, Vero cells, COS cells, HeLa cells, NT2 cells, MG63 cells, M24 metastatic melanoma cells, A375 metastatic melanoma cells, U251 glioma cells, D54 glioma cells, HT1080 fibrosarcoma cells, or the like.

In other specific embodiments, the cells are stem cells or progenitor cells, e.g., endothelial progenitor cells, mesenchymal stem cells or mesenchymal-like stem cells. In more specific embodiments, said mesenchymal stem cells or mesenchymal-like stem cells are from bone marrow, peripheral blood, umbilical cord blood, placental blood, placental tissue, umbilical cord tissue, Wharton's jelly, amniotic fluid, amnion, chorion, endometrium, adipose tissue, dental pulp, or dermis.

In certain embodiments the cells culturable on the ECM-comprising microcarriers are placental stem cells, e.g., CD10+, CD34-CD105+, CD100+ placental stem cells. In a more specific embodiment, said placental stem cells are CD34- and/or CD200+. In a specific embodiment, the placental stem cells are CD34-, CD10+, CD105+ and CD200+. In certain specific embodiments, the placental stem cells can express one or more of CD10, CD73, CD105, CD200, and/or OCT-4, and lack expression of one or more of CD34, CD38, CD45, and/or HLA-G. In certain other specific embodiments, the placental stem cells can also express HLA-ABC (MHC-1) and lack expression of HLA-DR. In another specific embodiment, the placental stem cells are CD200+ and HLA-G-. In another specific embodiment, the placental stem cells are CD73+, CD105+, and CD200+. In another specific embodiment, the placental stem cells are CD200+ and OCT-4+. In another specific embodiment, the placental stem cells are CD73+, CD105+ and HLA-G-. In another specific embodiment of any of the above embodiments, the stem cells secrete IL-6, IL-8 and/or MCP-1 (monocyte chemotactic protein-1) when cultured on the microcarriers.

### 3.1. Definitions

"Anchorage dependent cells" as used herein means cells that require attachment to a surface in order to proliferate in culture.

"Microcarrier" as used herein means ECM-containing particles, pellets or beads, or the like, to which attachment-dependent cells can attach and proliferate in culture. Microcarriers used in the methods provided herein are small enough to allow for use in suspension culture; are solid, or semi-solid; and have a mean (average) particle diameter (measured by the longest axis if the particles are not substantially spherical) of 10 micrometers to 1000 micrometers.

### 4. Brief Description of the Drawings

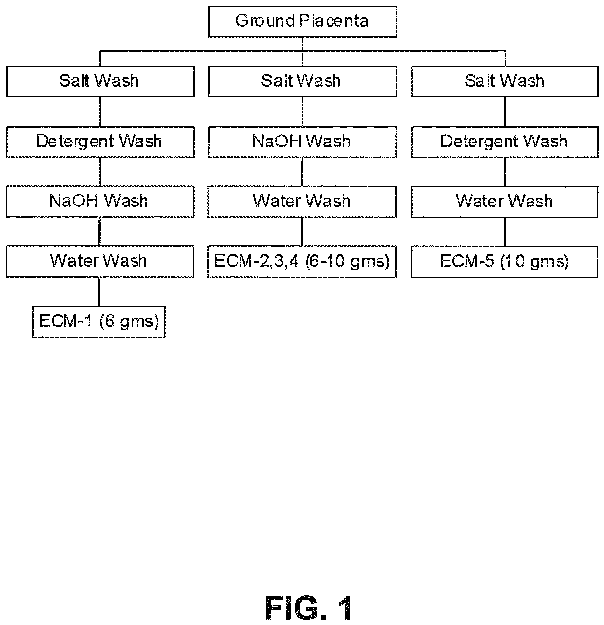

FIG. 1: Flow chart representation of methods for isolating extracellular matrix (ECM).

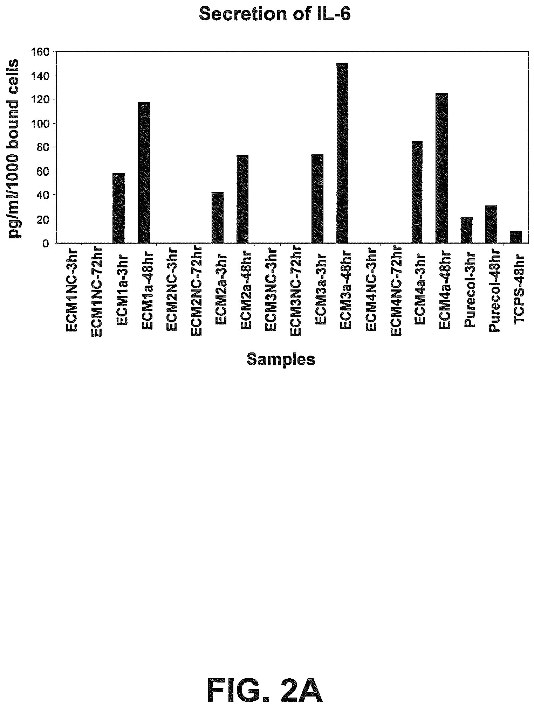

FIG. 2A: Secretion of IL-6 from placental stem cells grown on collagen composition made by different methods. Abscissa: Specific growth conditions by type of composition and time of growth of the cells on the composition. Ordinate: picograms per milliliter per 1000 ECM-bound cells. NC=no cells. Purecol=purified collagen. TCPS=tissue culture polystyrene.

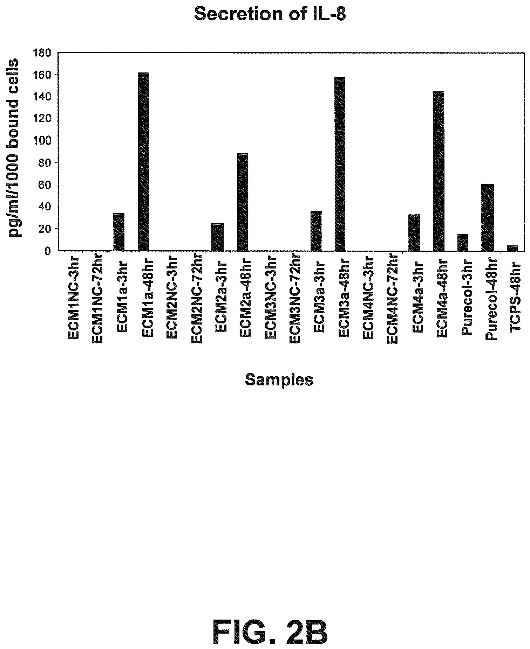

FIG. 2B: Secretion of IL-8 from placental stem cells grown on collagen composition made by different methods. Abscissa: Specific growth conditions by type of composition and time of growth of the cells on the composition. Ordinate: picograms per milliliter per 1000 ECM-bound cells. NC=no cells. Purecol=purified collagen. TCPS=tissue culture polystyrene.

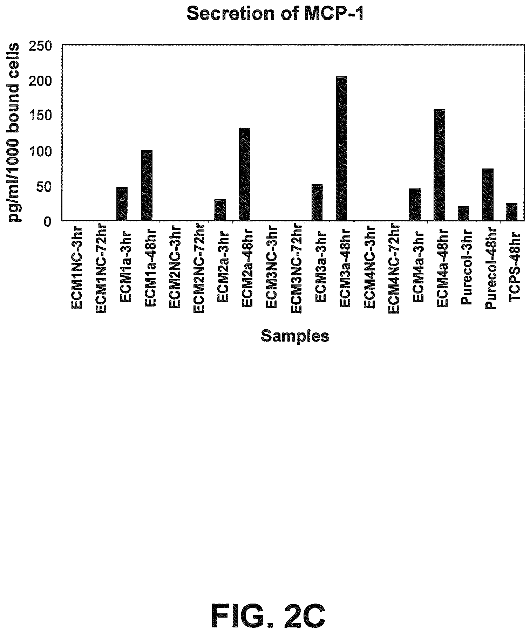

FIG. 2C: Secretion of MCP-1 from placental stem cells grown on collagen composition made by different methods. Abscissa: Specific growth conditions by type of composition and time of growth of the cells on the composition. Ordinate: picograms per milliliter per 1000 ECM-bound cells. NC=no cells. Purecol=purified collagen. TCPS=tissue culture polystyrene.

### 5. DETAILED DESCRIPTION

### 5.1. Microcarriers Comprising ECM, and Methods of Culturing Cells

Provided herein are microcarrers comprising extracellular matrix, e.g., human placental extracellular matrix (ECM). The microcarriers can be generally spherical, but may be any shape that facilitates culture of anchorage-dependent cells, e.g., ECM-adherent cells. The microcarriers can be essentially solid, or can be a mesh of fibers.

The microcarrers can consist of ECM, that is, be made entirely of ECM.

The microcarriers can consist essentially of ECM, that of, structurally the microcarriers are made of ECM, but the ECM comprises additional, non-structural components (e.g., bioactive molecules, crosslinking, or the like).

In certain other embodiments, the microcarriers comprise a second composition in addition to said ECM, for example, one or more of glass, dextran, DEAE-dextran, polystyrene, acrylamide, or collagen. In a specific embodiment, the ECM forms an exterior of said microcarrier, and said second composition forms an interior of said microcarrier. The ECM may cover a portion of, substantially the entire surface of, or the entire surface of, said microcarrier. In other words, the microcarrier may comprise a composition partly or completely covered by, or coated with, the ECM.

Beads that may be coated with ECM for use as microcarriers include, without limitation, e.g., CYTODEX 1®, CYTODEX 2®, CYTODEX 3® (GE Healthcare Life Sciences, Piscataway N.J.), HILLEX® (SoloHill Engineering, Inc., Ann Arbor, Mich.), CYTOLINE™ 1 or CYTOPORE™ beads (GE Healthcare Life Sciences), Biosilon (NUNC) beads, CULTISPHER® (Percell Biolytica) beads or the like. Suitable beads may also comprise cationic trimethyl ammonium attached to the surface to provide a positively charged surface to the microcarrier.

In any of the embodiments herein, the microcarrier is substantially in the shape of a sphere. In various specific embodiments, the microcarriers average, e.g., between 10 micrometers to 3000 micrometers in diameter; between 10-2000 micrometers in diameter, between 10-1000 micrometers in diameter, between 50 and 200 micrometers in diameter, between 100 and 250 micrometers in diameter; between 150 and 300 micrometers in diameter; between 200 and 350 micrometers in diameter; between 250 and 400 micrometers in diameter; between 200 and 1000 micrometers in diameter; between 300 and 1000 micrometers in diameter; between 400 and 1000 micrometers in diameter; between 500 and 1000 micrometers in diameter; between 600 and 1000 micrometers in diameter; between 700 and 1000 micrometers in diameter; between 800 and 1000 micrometers in diameter; or between 900 and 1000 micrometers in diameter.

Preferably, the microcarriers are of a density that allows for use of the microcarrier in bioreactor culture of cells. Excessive density may cause the microcarriers to settle out of the suspension, or tend to remain completely towards the bottom of the culture vessel, resulting in poor bulk mixing of the cells, culture medium and gaseous phases in the reactor, while a density that is too low may result in excessive floating of the microcarrier. Preferably, the density of the microcarriers comprising ECM is 1.02 to 1.15 g/cm³.

In certain embodiments, the microcarriers comprising ECM are porous. Porous microcarriers typically possess large cavities (relative to cell size) that are available for the growth of anchorage-dependent cells. These cavities increase the surface area greatly, and may protect cells from detrimental mechanical effects, such as shear stress, for example from mixing or from gas sparging.

The microcarrier surface may be smooth, or may be textured, e.g., to enhance cell attachment and proliferation. The microcarrier surface texture be achieved by techniques including, but not limited to, molding, casting, leeching and etching. The resolution of the features of the textured surface may be on the nanoscale. The textured surface may be used to induce a specific cell alignment on the microcarrier surface. The surface of the pores within the porous microcarriers may also be textured to enhance cell attachment and proliferation. Pore surface texture be achieved by techniques such as but not limited to molding, casting, leeching and etching.

The microcarrier surface may be plasma-coated to impart a specific charge to microcarrier surfaces, e.g., to enhance cell attachment and proliferation. The microcarriers may comprise, e.g., be coated with, a thermoresponsive polymer such as poly-N-isopropylacrylamide, or may have electromechanical properties. The microcarriers may possess a microcurrent, such as microcarriers with a particulate galvanic couple of zinc and copper that produces low levels of biologically relevant electricity. The microcarriers may be paramagnetic, such as paramagnetic calcium-alginate microcarriers.

The carrier particles may also contain a bioactive agent. The carrier particle may also contain a bioactive agent that may regulate the growth or function of cells or the tissue milieu these factors may include but are not limited to cytokines, e.g., fibroblast growth factor (FGF), erythropoietin (Epo), vascular endothelial cell growth factor (VEGF), platelet derived growth factor (PDGF), bone morphogenic proteins (BMPs), transforming growth factor (TGF), tumor necrosis factor (TNF alpha), epidermal growth factor (EGF), insulin-like growth factor (IGF), or others.

Microcarriers made substantially from, or solely from, ECM, may be made by producing ECM by any of the methods described elsewhere herein, substantially dehydrating the ECM, e.g., to 5% water content or less, and milling the ECM, e.g., using a jet mill until the desired average pellet size is obtained. Example systems that may be used to mill the dried ECM include, but are not limited to, the ROTO-JET® Fluid Bed Jet Milling/Processing platform from Fluid Energy Processing and Equipment Company (Telford, Pennsylvania). Similar systems from, e.g., Hosokawa Micron Powder Systems (Summit, New Jersey), Kemutec (Bristol, Pennsylvania), K-Tron Industries (Pitman, New Jersey), or the like, may be used, as well. ECM particle sizes may be checked/validated using, e.g., a ROTO-SIZER® Classification/Processing System from Fluid Energy Processing and Equipment Company (Telford, Pennsylvania).

Cells, e.g., the placental stem cells described elsewhere herein, may be cultured using in the ECM-containing microcarriers in a bioreactor. The bioreactor may be, for example, a batch reactor, a fed batch reactor, or a continuous stirred-tank reactor. The bioreactor may be any useful size, e.g., 1L, 2L, 3L, 4L, 5L, 6L, 7L, 8L, 9L, 10L, 15L, 20L, 25L, 30L, 35L, 40L, 45L, 50L, 60L, 70L, 80L, 90L, 100L, 120L, 140L, 160L, 180L, 200L, 250L, 300L, 350L, 400L, 450L, 500L, 550L, 600L, 650L, 700L, 750L, 800L, 850L, 900L, 950L, 1000L, 1100L, 1200L, 1300L, 1400L, 1500L or more, or 1L to 10L, 5L to 20L, 10L to 30L, 15L to 40L, 20L to 50L, 30L to 60L, 40L to 70L, 50L to 80L, 60L to 90L, 70L to 100L, 80L to 120L, 90L to 140L, 100L to 150L, 120L to 160L, 140L to 180L, 150L to 200L, 175L to 250L, 200L to 300L, 250L to 350L, 300L to 400L, 350L to 450L, 400L to 500L, 450L to 550L, 500L to 600L, 550L to 650L, 600L to 650L, 600L to 700L, 650L to 750L, 700L to 800L, 750L to 850L, 800L to 900L, 850L to 950L, 900L to 1000L, 1000L to 1100L, 1100L to 1200L, 1200L to 1300L, 1300L to 1400L, or 1400L to 1500L.

Cells used for microcarrier culture may be initially cultured, e.g., from initial isolation, or from thawed cryopreserved cell stocks, e.g., by the methods described for placental stem cells below. Preferably, the cells constitute a population in suspension culture, substantially free of aggregates and clumps (e.g., fewer than 10%, 9%, 9%, 7%, 6%, 5%, 4%, 3%, 2%, 1%, 0.5% or 0.2% of the cells are in cell clumps or aggregates). If the cells are to be obtained from plate culture, the cells can be made into a single-cell suspension by disassociating the cells from the plate using, e.g., trypsin, ACCUTASE®, or the like. Extraneous protein may be removed from plate cell cultures by, e.g., rinsing with Ca/MG-free phosphate-buffered saline (PBS), e.g., Dulbecco's PBS (DPBS) prior to detachment. Detachment can take place at room temperature (e.g., about 23°C) to about 37°C, or at any temperature in between. Detachment can proceed from 20 minutes to 2 hours, depending upon cell type.

Cells and microcarriers may be combined in liquid suspension according to a ratio of number of cells to a surface area of the microspheres, in the aggregate. For example, the number of square centimeters of a particular batch of ECM-containing microcarriers is calculated.

Cells can be combined with the ECM-containing microcarriers at a ratio of, for example, 1.0 x 10³ cells/cm², 2.0 x 10³ cells/cm², 3.0 x 10³ cells/cm², 4.0 x 10³ cells/cm², 5.0 x 10³ cells/cm², 6.0 x 10³ cells/cm², 7.0 x 10³ cells/cm², 8.0 x 10³ cells/cm², 9.0 x 10³ cells/cm², 1.0 x 10³ cells/cm², 2.0 x 10³ cells/cm², 3.0 x 10³ cells/cm², 4.0 x 10³ cells/cm², 5.0 x 10³ cells/cm², 6.0 x 10³ cells/cm², 7.0 x 10³ cells/cm², 8.0 x 10³ cells/cm², 9.0 x 10³ cells/cm², 1.0 x 10⁴ cells/cm², 1.0 x 10⁴ cells/cm², 2.0 x 10⁴ cells/cm², 3.0 x 10⁴ cells/cm², 4.0 x 10⁴ cells/cm², 5.0 x 10⁴ cells/cm², 6.0 x 10⁴ cells/cm², 7.0 x 10⁴ cells/cm², 8.0 x 10⁴ cells/cm², 9.0 x 10⁴ cells/cm², 1.0 x 10⁵ cells/cm², or more. Preferably, the cells are inoculated at between about 1.0 x 10⁴ cells/cm² and 5.0 x 10⁴ cells/cm², 2.0 x 10⁴ cells/cm² and 6.0 x 10⁴ cells/cm², or 2.0 x 10⁴ cells/cm² and 3.0 x 10⁴ cells/cm².

Total number of cells to inoculate into a culture container comprising the ECM-containing microcarriers can be determined by multiplying the total number of square centimeters of ECM-containing microcarrier surface by the number of cells per square centimeter of surface. Typically, the ECM-containing microcarriers and cells are combined in medium that is gently agitated so as to maintain a suspension of both, while maximizing cellmicrocarrier attachment and minimizing cell-cell attachment and clumping. Cells should be given a sufficient time to attach to the microcarriers, e.g., 10, 15, 20, 25, 30, 35, 40, 45, 50, 55, 60, 70, 75, 80, 85, 90, 95, 100, 105, 110, 115, 120, 125, 130, 135, 140, 145, 150, 155, 160, 165, 170, 175, or 180 minutes, or 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or 36 hours, or longer. Samples of homogeneous microcarrier-cell mixture can be assessed visually at the end of a given time point, or at various times during attachment, to determine when substantially all cells (e.g., greater than 90%, 95%, 98% or 99% of cells) have attached to the microcarriers.

For cells that are adapted to a culture environment free of animal protein, or for cell lines having low plating efficiency, the above protocol can be modified by constant stirring for 15, 16, 17, 18, 19, 20 or 21 hours after contacting the cells with the ECM-containing microcarriers, followed by intermittent stirring once cells have attached and begun to spread along the microcarrier. For example, intermittent stirring may comprise stirring for 1 minute followed by a 30 minute rest during which no stirring takes place, designated for this context a 1:30 ratio. Other stirring time/rest time ratios that may be used include, e.g., 1:20, 2:20, 3:20, 4:20, 5:20, 6:20, 7:20, 8:20, 9:20, 10:20, 11:20, 12:20, 13:20, 14:20, 15:20, 16:20, 17:20, 18:20, 19:20, 20:20, 1:30, 2:30, 3:30, 4:30, 5:30, 6:30, 7:30, 8:30, 9:30, 10:30, 11:30, 12:30, 13:30, 14:30, 15:30, 16:30, 17:30, 18:30, 19:30, 20:30, 21:30, 22:30, 23:30, 24:30, 25:30, 26:30, 27:30, 28:30, 29:30 or 30:30, or the like. After the cells have spread, constant stirring may be resumed.

During culture of cells on microcarriers, the cells may be cultured in any medium that is acceptable for the type of cell being cultured.

In specific embodiments, for example, adherent placental stem cells can be cultured in any medium recognized in the art as acceptable for the culture of stem cells, e.g., mesenchymal-like stem cells. In certain embodiments, the culture medium comprises serum, e.g., human serum or bovine calf serum/fetal calf serum. In certain other embodiments, the culture medium is serum-free. Placental stem cells can be cultured in, for example, DMEM-LG (Dulbecco's Modified Essential Medium, low glucose)/MCDB 201 (chick fibroblast basal medium) containing ITS (insulin-transferrin-selenium), LA+BSA (linoleic acid-bovine serum albumin), dextrose, L-ascorbic acid, PDGF, EGF, IGF-1, and penicillin/streptomycin; DMEM-HG (high glucose) comprising 10% fetal bovine serum (FBS); DMEM-HG comprising 15% FBS; IMDM (Iscove's modified Dulbecco's medium) comprising 10% FBS, 10% horse serum, and hydrocortisone; M199 comprising 10% FBS, EGF, and heparin; α-MEM (minimal essential medium) comprising 10% FBS, GLUTAMAX™ and gentamicin; DMEM comprising 10% FBS, GLUTAMAX™ and gentamicin, etc. In one embodiment, the medium is DMEM-LG/MCDB-201 comprising 2% FBS, ITS, LA+BSA, dextrose, L-ascorbic acid, PDGF, EGF, and penicillin/streptomycin. Other media in that can be used to culture placental stem cells include DMEM (high or low glucose), Eagle's basal medium, Ham's F10 medium (F10), Ham's F-12 medium (F12), Iscove's modified Dulbecco's medium, Mesenchymal Stem Cell Growth Medium (MSCGM), Liebovitz's L-15 medium, MCDB, DMEM/F12, RPMI 1640, advanced DMEM (Gibco), DMEM/MCDB201 (Sigma), and CELL-GRO FREE.

The culture medium, e.g., for culture of adherent placental stem cells, can be supplemented with one or more components including, for example, serum (e.g., fetal bovine serum (FBS), preferably about 2-15% (v/v); equine (horse) serum (ES); human serum (HS)); beta-mercaptoethanol (BME), preferably about 0.001% (v/v); one or more growth factors, for example, platelet-derived growth factor (PDGF), epidermal growth factor (EGF), basic fibroblast growth factor (bFGF), insulin-like growth factor-1 (IGF-1), leukemia inhibitory factor (LIF), vascular endothelial growth factor (VEGF), and erythropoietin (EPO); amino acids, including L-valine; and one or more antibiotic and/or antimycotic agents to control microbial contamination, such as, for example, penicillin G, streptomycin sulfate, amphotericin B, gentamicin, and nystatin, either alone or in combination.

Cells may be harvested from the microcarriers using an enzyme such as trypsin, ACCUTASE®, or other suitable enzyme know to those in the art. For example, the cell-containing microcarriers can be collected in a volume of medium, and allowed to settle, followed by decanting excess medium. The remaining cell-containing microcarriers are preferably washed 2-5 times with a saline solution, e.g., Ca/Mg-free phosphate-buffered saline, e.g., Dulbecco's PBS (DPBS). The saline solution should not be applied to the microcarriers so as to promote dislodging of the cells. A rinse cycle may proceed, e.g., with stirring at 30-50RPM at 23°C to 37°C for 5-20 minutes, e.g., 10-15 minutes. After rinsing, the cell-containing microcarriers can be contacted with the enzyme to dissociate the cells. The concentration of enzyme used can range from, e.g., 0.01% to 1.0% w/v, e.g., 0.05% to 0.25% w/v. The cell-containing microcarriers can be exposed to the enzyme for, e.g., 1, 2, 3, 4, 5, 6, 7, 8, 9, 10, 11, 12, 13, 14, 15, 16, 17, 18, 19 or 20 minutes, or longer. The amount of enzyme used, as well as the time for dissociation, can be assayed visually at various time points; too little dissociation time or too little enzyme results in too few cells being dissociated (e.g., as visualizable under a microscope), and too much dissociation time or enzyme results in cell death. Cell death can be assessed by standard assays, e.g., trypan blue, MTS assay, MTT assay, or the like.

Placental stem cells can be cultured in standard tissue culture conditions, e.g., in tissue culture dishes or multiwell plates. Placental stem cells can also be cultured using a hanging drop method. In this method, placental stem cells are suspended at about 1 X 10⁴ cells per mL in about 5 mL of medium, and one or more drops of the medium are placed on the inside of the lid of a tissue culture container, e.g., a 100 mL Petri dish. The drops can be, e.g., single drops, or multiple drops from, e.g., a multichannel pipetter. The lid is carefully inverted and placed on top of the bottom of the dish, which contains a volume of liquid, e.g., sterile PBS sufficient to maintain the moisture content in the dish atmosphere, and the stem cells are cultured.

Once an isolated placental stem cell, or isolated population of stem cells comprising placental stem cells (e.g., a stem cell or population of stem cells separated from at least 50% of the placental cells with which the stem cell or population of stem cells is normally associated in vivo) is obtained, the placental stem cells or population of cells can be proliferated and expanded in vitro. For example, placental stem cells can be cultured in tissue culture containers, e.g., dishes, flasks, multiwell plates, or the like, for a sufficient time for the stem cells to proliferate to 70-90% confluence, that is, until the stem cells and their progeny occupy 70-90% of the culturing surface area of the tissue culture container.

Placental stem cells can be seeded in culture vessels at a density that allows cell growth. For example, the cells may be seeded at low density (e.g., about 1,000 to about 5,000 cells/cm²) to high density (e.g., about 50,000 or more cells/cm²). In a preferred embodiment, the cells are cultured at about 0 to about 5 percent by volume CO₂ in air. In some preferred embodiments, the cells are cultured at about 2 to about 25 percent O₂ in air, preferably about 5 to about 20 percent O₂ in air. The cells preferably are cultured at about 25°C to about 40°C, preferably 37°C. The cells are preferably cultured in an incubator. The culture medium can be static or agitated, for example, using a bioreactor. Placental stem cells may be grown under low oxidative stress (e.g., with addition of glutathione, ascorbic acid, catalase, tocopherol, N-acetylcysteine, or the like).

### 5.2. Extracellular Matrix Compositions

ECM for use in the microcarriers, and methods of cell culture using the microcarriers, provided herein above may, in certain embodiments, be obtained from a mammalian source, e.g., a human, bovine, ovine, sheep, rat source. The ECM compositions may be obtained from a marsupial, e.g., a kangaroo. In certain embodiments, the ECM is obtained from a nonmammalian source, e.g., the ECM is obtained from fish.

The ECM can be obtained from any portion of the source. In certain embodiments, the ECM can be obtained from, e.g., bovine skin, calf skin, rat tail, kangaroo tail or fish skin. In particular embodiments, the ECM is obtained from placenta, e.g., bovine placental ECM, ovine placental ECM or human placental ECM.

A principal component of the ECM, e.g., human placental ECM, is collagen. The collagen can be any type of collagen known to those of skill in the art or a mixture of such collagens. In certain embodiments, the collagen is in the form of a collagen composition that comprises one or more types of collagen. Particular collagens include type I collagen, type II collagen, type III collagen and type IV collagen. In certain embodiments, ECM comprises particular amounts of these collagens. In a particular embodiment, the ECM comprises a substantial amount of type I collagen while also being enriched in type IV collagen. In certain embodiments, the ECM comprises between 1% and 15% type IV collagen, between 2% and 13% type IV collagen, between 3% and 12% type IV collagen or between 4% and 11% type IV collagen by dry weight. In certain embodiments, the ECM comprises at least 75%, at least 80%, at least 85%, at least 90%, at least 95%, or at least 99% type I collagen by dry weight. In certain embodiments, the ECM comprises between 70% and 95% type I collagen, between 74% and 92% type I collagen or between 80% and 90% type I collagen by dry weight. In certain embodiments, the ECM comprises type III collagen, for instance up to 1%, up to 2%, up to 3%, up to 4%, up to 5%, up to 6% or up to 7% type III collagen by dry weight. In certain embodiments, the ECM comprises between 2% and 15% type IV collagen, between 70% and 95% type I collagen and up to 6% type III collagen, by dry weight.

In certain embodiments, the ECM comprises one or more extracellular matrix proteins or components in addition to collagens. For example, in specific embodiments, the ECM comprises one or more of fibronectin, laminin, elastin, and/or glycosaminoglycan. In other specific embodiments, the ECM comprises no detectable fibronectin, or no detectable laminin, or no detectable laminin or fibronectin. In another specific embodiment, the ECM comprises detectable amounts of fibronectin and laminin. In another specific embodiment, the ECM comprises about 5% or more elastin by dry weight. In another specific embodiment, the ECM comprises about 10% or more elastin by dry weight. In another specific embodiment, the ECM comprises no more than about 5% elastin by dry weight.

In certain embodiments, the ECM comprises less than 1%, less than 0.5%, less than 0.1%, less than 0.05%, or less than 0.01% laminin, or comprise between 0.01%-0.05%, 0.05%-0.1%, 0.1%-0.5%, or 0.5%-1.0% laminin, as compared to the total amount of protein in the composition (e.g., by dry weight). In certain embodiments, the ECM comprises less than 1%, less than 0.5%, less than 0.1%, less than 0.05%, or less than 0.01% fibronectin, or comprise between 0.01%-0.05%, 0.05%-0.1%, 0.1%-0.5%, or 0.5%-1.0% fibronectin, as compared to the total amount of protein in the composition (e.g., by dry weight). In certain embodiments, the ECM comprises about 1%, 2%, 3%, 4%, 5%, 6%, 7%, 8%, 9%, 10%, 11%, 12%, 13%, 14%, or 15% elastin or between 1-5%, 5-10%, or 10-15% elastin, and comprises less than 1%, less than 0.5%, less than 0.1%, less than 0.05%, or less than 0.01% laminin, or comprises between 0.01%-0.05%, 0.05%-0.1%, 0.1%-0.5%, or 0.5%-1.0% laminin; and/or less than 1%, less than 0.5%, less than 0.1%, less than 0.05%, or less than 0.01% fibronectin, or comprises between 0.01%-0.05%, 0.05%-0.1%, 0.1%-0.5%, or 0.5%-1.0% fibronectin, as compared to the total amount of protein in the composition (e.g., by dry weight). In a specific embodiment, the ECM comprises greater than 80% collagen, between 10-15% elastin, less than 0.01% laminin, and less than 0.01% fibronectin, as compared to the total amount of protein in the composition (e.g., by dry weight).

In certain embodiments, the ECM utilized in the microcarriers and methods provided herein can be obtained by one of the processes described below.

In certain embodiments, at least the collagen in the ECM is cross-linked, e.g., with a cross-linker. In certain embodiments, the cross-linker is glutaraldehyde. See, e.g., U.S. Pat. Nos. 4,852,640, 5,428,022, 5,660,692 and 5,008,116, and in McPherson et al., 1986, J. Biomedical Materials Res. 20:79-92, the contents of which are hereby incorporated by reference in their entirety. Further exemplary cross-linkers and methods of cross-linking collagen are described in U.S. Pat. Nos. 5,880,242 and 6,117,979 and in Zeeman et al., 2000, J Biomed Mater Res. 51(4):541-8, van Wachem et al., 2000, J Biomed Mater Res. 53(1):18-27, van Wachem et al., 1999, J Biomed Mater Res. 47(2):270-7, Zeeman et al., 1999, J Biomed Mater Res. 46(3):424-33, Zeeman et al., 1999, Biomaterials 20(10):921-31, the contents of which are hereby incorporated by reference in their entireties.

In further embodiments collagen in the ECM is cross-linked with 1,4-butanediol diglycidyl ether. In further embodiments collagen in the ECM is cross-linked with genipin, a non-toxic, naturally occurring crosslinking agent. Genipin can be obtained from its parent compound, geniposide, which may be isolated from the fruits of *Gardenia jasminoides.* Genipin may be obtained commercially from Challenge Bioproducts Co., Ltd., 7 Alley 25, Lane 63, TzuChiang St. 404 Taichung Taiwan R.O.C., Tel 886-4-3600852. The use of genipin as a crosslinking reagent is described extensively in U.S. Patent Application Publication No. 2003/0049301, the contents of which are hereby incorporated by reference in their entirety.

The collagen in the ECM can be cross-linked with a single cross-linker or with a mixture of cross-linkers. In certain embodiments, ECM comprises base-treated, detergent treated human placental collagen cross-linked with glutaraldehyde.

The collagen in the ECM can be cross-linked with any enzyme-mediated crosslinking technique known to those of skill in the art. For instance, the collagen in the ECM can be cross-linked by transglutaminase, e.g., by the methods described, for example, in Orban et al., 2004, J. Biomedical Materials Res. 68(4):756-62.

### 5.3. Processes for Preparation of ECM

In certain embodiments, the ECM may be prepared from human placenta. The placental tissue can be from any part of the placenta including the amnion, whether soluble or insoluble or both, the chorion, the umbilical cord or from the entire placenta. In certain embodiments, the ECM is prepared from whole human placenta without the umbilical cord. In certain other embodiments, the ECM is prepared from whole placenta without the amniotic membrane or umbilical cord.

The placenta from which ECM is obtained is preferably taken as soon as possible after normal delivery, or after cesarean section delivery, of a normal healthy infant. Advantageously, the placenta is collected under aseptic conditions. The placenta, in certain embodiments, is stored for 48 hours from the time of delivery prior to any further treatment. In other embodiments, the placenta is stored for up to 5 days from the time of delivery prior to any further treatment.

The placenta and optionally umbilical cord can be transported from the delivery or birthing room to another location, e.g., a laboratory, for further processing. The placenta can be transported in a sterile, transport device such as a sterile bag or a container, which is optionally thermally insulated. In some embodiments, the placenta is stored at room temperature until further treatment. In other embodiments, the placenta is refrigerated until further treatment, i.e., stored at a temperature of about 2°C to 8°C. The placenta may be stored under sterile conditions for up to 5 days before further treatment. The placenta is preferably handled and processed under aseptic conditions, as known to persons skilled in the art, e.g., in a laboratory can be equipped with an HEPA filtration system (as defined by clean room classification, having a class 1000 or better).

The placenta is preferably exsanguinated, i.e., completely drained of the cord blood remaining after birth, prior to obtaining the ECM. In some embodiments, the placenta is 70% exsanguinated, 80% exsanguinated, 90% exsanguinated, 95% exsanguinated or 99% exsanguinated.

The expectant mother may be screened for known pathogens prior to the time of birth, using standard techniques known to one skilled in the art, for communicable diseases including but not limited to, HIV, HBV, HCV, HTLV, syphilis, CMV, and other viral pathogens known to contaminate placental tissue, e.g., following FDA regulations. The expectant mother may be screened (e.g., a blood sample is taken for diagnostic purposes) within one month of birth, particularly within two weeks of birth, within one week of birth, or at the time of birth. Preferably, only tissues collected from donors who tested negative or non-reactive to the above-mentioned pathogens are used to produce ECM. Advantageously, a thorough paternal and medical and social history of the donor of the placental membrane is obtained, including for example, a detailed family history.

In certain embodiments, the donor is screened using standard serological and bacteriological tests known to persons skilled in the art. For example, donor screening can encompass using standard antigen-detection techniques known to one skilled in the art using, e.g., antibody screen (ATY); alanine amino transferase screening (ALT); Hepatitis Core Antibody (nucleic acid and ELISA); Hepatitis B Surface Antigen; Hepatitis C Virus Antibody; HIV-1 and HIV-2; HTLV-1 and HTLV-2; Syphilis test (RPR); CMV antibody test; and/or Hepatitis C and HIV test. The assays used may be nucleic acid based assays or ELISA based assays as known to one skilled in the art.

Blood from the umbilical cord of the newborn using standard techniques known to one skilled in the art (See, e.g., Cotorruelo et al., 2002, Clin. Lab. 48(5 6):271 81; Maine et al., 2001, Expert Rev. Mol. Diagn., 1(1):19 29; Nielsen et al., 1987, J. Clin. Microbiol. 25(8):1406-10). In one embodiment, blood from the umbilical cord of the newborn is tested for bacterial pathogens (including but not limited to gram positive and gram negative bacteria) and/or fungi using standard techniques known to persons skilled in the art. The blood type and Rh factor of the blood of the umbilical cord of the newborn can be determined using standard techniques known to those skilled in the art. In another embodiment, complete blood count (CBC) with differential is obtained from the blood from the umbilical cord of the newborn using standard methods known to one skilled in the art. In yet another embodiment, an aerobic bacterial culture is taken from the blood from the umbilical cord of the newborn, using standard methods known to one skilled in the art. Only tissues collected from donors that have a CBC within a normal limit (e.g., no gross abnormality or deviation from the normal level), test negative for serology and bacteriology, and test negative or non-reactive for infectious disease and contamination are used to produce the ECM.

Once the human placental tissue is obtained, it can be treated according to the following steps in order to prepare the ECM. Although the following steps are presented in sequential order, one of skill in the art will recognize that the order of several steps can be interchanged. It is assumed that techniques readily apparent to those of skill in the art such as buffer exchange, precipitation, centrifugation, resuspension, dilution and concentration of protein compositions need not be explained in detail. Exemplary preparation is described in the examples below.

Any portion of the placenta, or the entire placenta, can be used in the processes described herein. In certain embodiments, ECM is prepared from whole placenta, or from chorionic or amnionic portions of the placenta.

The umbilical cord may be separated from the placental disc, and the amniotic membrane may be separated from the chorionic membrane. The amniotic membrane may be separated from the chorionic membrane prior to cutting the placental membrane. Separation of the amniotic membrane from the chorionic membrane can be done starting from the edge of the placental membrane, and can be separated from the chorionic membrane using blunt dissection, e.g., with gloved fingers. Following separation of the amniotic membrane from the chorionic membrane and placental disc, the umbilical cord stump may be cut, e.g., with scissors, and detached from the placental disc. In certain embodiments, when separation of the amniotic and chorionic membranes is not possible without tearing the tissue, the amniotic and chorionic membranes may be cut from the placental disc as one piece and then peeled them apart.

The amniotic membrane, chorionic membrane or whole placenta can be stored prior to use in the methods described herein. Exemplary storage techniques are described in U.S. Patent Application Publication Nos. 2004/0048796 and 2003/0187515, the contents of which are hereby incorporated by reference in their entireties.

The placental tissue may be decellularized prior to obtaining the ECM. The placental tissue can be decellularized according to any technique known to those of skill in the art, e.g., techniques described in U.S. Patent Application Publication Nos. 2004/0048796 and 2003/0187515, the contents of which are hereby incorporated by reference in their entireties.

In certain embodiments, the placental tissue is subjected to an osmotic shock. Although not intending to be bound by any particular theory of operation, it is believed that the osmotic shock can burst cells in the tissue and thereby facilitating removal of cells, cellular components and blood components. Osmotic shock can be in addition to any clarification step or it can be the sole clarification step.

The osmotic shock can be carried out in any osmotic shock conditions known to those of skill in the art. Such conditions include incubating the tissue in solutions of high osmotic potential, or of low osmotic potential or of alternating high and low osmotic potential. The high osmotic potential solution can be any high osmotic potential solution known to those of skill in the art such as a solution comprising one or more of NaCl (e.g., 0.2M to 1.0M), KCl (e.g., 0.2M to 1.0M or 2.0M), ammonium sulfate, a monosaccharide, a disaccharide (e.g., 20% sucrose), a hydrophilic polymer (e.g., polyethylene glycol), glycerol, etc. In certain embodiments, the high osmotic potential solution is a sodium chloride solution. In some embodiments, the sodium chloride solution is at least 0.25M, 0.5M, 0.75M, 1.0M, 1.25M, 1.5M, 1.75M, 2M, 2.25M or 2.5M NaCl. In some embodiments, the sodium chloride solution is about 0.25M to 5M, about 0.5M to 4M, about 0.75M to 3M, or about 1.0M to 2.0M NaCl.

The low osmotic potential solution can be any low osmotic potential solution known to those of skill in the art, such as water, for example water deionized according to any method known to those of skill. In some embodiments, the osmotic shock solution comprises water with an osmotic shock potential less than that of 50 mM NaCl.

In certain embodiments, the osmotic shock is accomplished using a sodium chloride solution followed by a water solution. In various embodiments, the sodium chloride solution is at least 0.5M NaCl, at least 0.75M NaCl, at least 1.0M NaCl, at least 1.5M NaCl, or at least 2.0M NaCl. In certain embodiments, one 0.5M NaCl treatment is followed by a water wash. In certain embodiments, two consecutive 0.5M NaCl treatments are followed by a water wash. In certain embodiments, one 2M NaCl treatment is followed by a water wash. These sequences can be repeated according to the judgment of one of skill in the art.

In certain embodiments, the ECM-containing composition resulting from the osmotic shock can be incubated with a detergent. The detergent can be any detergent known to those of skill in the art to be capable of disrupting cellular or subcellular membranes. In certain embodiments, the detergent is ionic. For instance, in certain embodiments, the detergent is deoxycholate, deoxycholic acid or sodium dodecylsulfate. In certain embodiments, the detergent is zwitterionic. In certain embodiments, the detergent is nonionic. For instance, in certain embodiments, the detergent can be a TWEEN® detergent, such as TWFFN®-20, or a Triton X detergent, such as Triton X 100. The collagen composition can be contacted with the detergent under conditions judged by one of skill in the art to be suitable for removing unwanted components from the composition. Exemplary conditions are provided in the working examples below.

The detergent treatment can be carried out at any temperature according to the judgment of those of skill in the art. In certain embodiments, the detergent treatment is carried out at about 0°C to 30°C., about 5°C to 25°C, about 5°C to 20°C, or about 5°C to 15°C. In certain embodiments, the detergent treatment is carried out at about 0°C, about 5°C, about 10°C, about 15°C, about 20°C, about 25°C, or about 30°C. In particular embodiments, the detergent treatment is carried out at about 5°C to 15°C.

The detergent treatment can be carried out for a suitable time according to the judgment of those of skill in the art. In certain embodiments, the detergent treatment can be carried out for about 1-24 hours, about 2-20 hours, about 5-15 hours, about 8-12 hours, or about 2-5 hours.

In some embodiments, the ECM is not treated with a base. In other embodiments, the ECM composition resulting from the detergent treatment can be optionally treated one or more times with a base, e.g., by washing the ECM in a basic solution. In certain embodiments, the base removes endotoxins and/or viral particles. Exemplary bases for the basic treatment include biocompatible bases, volatile bases or bases known to those of skill in the art to be easily and safely removable from the ECM. The base can be any organic or inorganic bases known to those of skill in the art at a concentration of, for example, 0.2M to 1.0M. In certain embodiments, the base is ammonium hydroxide, potassium hydroxide or sodium hydroxide, e.g., an ammonium hydroxide solution, potassium hydroxide solution or sodium hydroxide solution. The sodium hydroxide solution can be 0.1M NaOH, 0.25M NaOH, 0.5M NaOH, or 1M NaOH. In particular embodiments, the basic treatment is carried out in 0.1M or 0.5M NaOH.

The base treatment can be carried out at any temperature according to the judgment of those of skill in the art. In certain embodiments, the basic treatment is carried out at about 0°C to 30°C, about 5°C to 25°C, about 5°C to 20°C, or about 5°C to 15°C. In certain embodiments, the basic treatment is carried out at about 0°C, about 5°C, about 10°C, about 15°C, about 20°C, about 25°C, or about 30°C. In particular embodiments, the basic treatment is carried out at about 5°C to 15°C.

The base treatment can be carried out for a suitable time according to the judgment of those of skill in the art. In certain embodiments, the basic treatment can be carried out for about 1-24 hours, about 2-20 hours, about 5-15 hours, about 8-12 hours, or about 2-5 hours.

Variations of the detergent and NaOH wash steps can be used to generate a number of variations of the ECM. For example, in certain embodiments, the ECM can be treated with about 0.1M, 0.2M, 0.3M, 0.4M, or about 0.5M NaOH over about 3, 4, 5, 6, 7, 8, 9, 10, 11, 12, 13, 14, 15, 16, 17, 18, 19, 20, 21, 22, 23, or about 24 hours.

In certain other embodiments, the ECM is produced without treatment by a base. Where the process is applied to placental tissue, omission of a base treatment step typically results in ECM comprising relatively higher amounts of elastin, fibronectin and/or laminin than the ECM produced with inclusion of the base treatment.

Preferably, any or all of the above steps are carried out under sterile conditions. In particular embodiments, the basic treatment, and all subsequent steps, are carried out under sterile conditions. In further embodiments, any collagen composition prepared according to the methods described herein can be further sterilized according to techniques apparent to one of skill in the art.

In certain embodiments, a method of preparing the ECM, e.g., comprises tissue, e.g., placental tissue, to the osmotic shock, freeze dry, detergent treatment, water wash, freeze dry, base treatment, water wash and freeze dry steps described above, e.g., carried out in order. In certain embodiments, the detergent is 1% deoxycholate. In certain embodiments, the base treatment is 0.5 N NaOH for four hours. In certain embodiments, the first water wash is repeated (two total washes). In certain embodiments, the second water wash is repeated twice (three total washes). In certain embodiments, the detergent is 1% deoxycholate, the basic treatment is 0.5 N NaOH for four hours, the first water wash is repeated (two total washes) and the second water wash is repeated twice (three total washes). In certain embodiments, such a process can provide a composition comprising about 0.59% glycosaminoglycans, about 3.5% elastin, little or no fibronectin and little or no laminin.

In certain embodiments, ECM is obtained by subjecting tissue, e.g., placental tissue, to osmotic shock, base treatment, and water wash, e.g., as described above. In certain embodiments, these steps are carried out in order. In certain embodiments, the base treatment is 0.5 N NaOH for four hours. In certain embodiments, the ECM resulting from such preparation comprises about 0.28% to about 0.38% glycosaminoglycans, about 3.2% to about 4.7% elastin, little or no (e.g., less than 1%) fibronectin and little or no (e.g., less than 1%) laminin, by dry weight.

In certain embodiments, ECM is obtained by subjecting tissue, e.g., placental tissue, to osmotic shock, detergent treatment and water wash steps, e.g., as described above. In certain embodiments, these steps are carried out in order. In certain embodiments, the detergent is 1% deoxycholate. In certain embodiments, such a process can provide a composition comprising about 0.4% glycosaminoglycans, about 12% elastin, about 0.6% fibronectin and about 0.16% laminin.

### 5.4. Optional Further Treatment

In certain embodiment the ECM usable in the microcarriers and culture methods provided herein comprises telopeptide collagen. In certain embodiments, the ECM comprises atelopeptide collagen. In yet other embodiments, an ECM composition comprising telopeptide collagen can be used as a source for an atelopeptide collagen composition. The atelopeptide collagen composition can be used for any purpose apparent to those of skill in the art for atelopeptide collagen.

In such embodiments, ECM can be contacted with an enzyme capable or partially or completely removing telopeptides from the collagen contained therein. The enzyme can be any proteolytic enzyme known to those of skill in the art that is capable of removing telopeptides from collagen. In certain embodiments, the enzyme is pepsin or papain. Generally, the enzyme is contacted with the ECM composition under conditions suitable for removal of telopeptide known to those of skill in the art. Methods of treating ECM compositions with enzymes to remove telopeptides are described in, e.g., U.S. Pat. Nos. 4,511,653, 4,582,640, 5,436,135 and 6,548,077, the contents of which are hereby incorporated by reference in their entireties.

In certain embodiments, the ECM composition is contacted with pepsin at about 15°C to 40°C, about 20°C to 35°C, about 25°C to 30°C, about 20°C to 30°C, or about 23°C to 27°C. In particular embodiments, the ECM composition is contacted with pepsin at about 23°C to 27°C for a time sufficient to remove telopeptide. The ECM composition may be contacted with the enzyme for a time sufficient to remove telopeptides from the collagen therein. In certain embodiments, for example, the ECM is contacted with pepsin for at least 5, 10, 15, 20, 25 or 30 hours. In certain embodiments, the ECM is contacted with pepsin for about 5 to 30 hours, about 10 to 25 hours or about 20 to 25 hours. In certain embodiments, the ECM is contacted with pepsin for about 8, 16, 24 or 32 hours.

The collagen composition is, in certain embodiments, contacted with the enzyme in an amount suitable to remove substantially all telopeptide from the ECM. In some embodiments, about 0.1 g, 0.5 g, 1.0 g, 2.0 g or 5.0 g pepsin per kg ECM dry weight is contacted with the ECM. In other embodiments, about 0.1 g, 0.5 g, 1.0 g, 2.0 g or 5.0 g pepsin/placenta is contacted with the ECM. In certain embodiments, the collagen composition is contacted with about 0.1 to 10.0 g/L, about 0.5 to 5/L, about 1 to 2.5 g/L, or about 0.5 1.5 g/L pepsin. In some embodiments, the collagen composition is contacted with about 0.1 g/L, about 0.2 g/L, about 0.5 g/L, about 1.0 g/L, about 2.0 g/L, 5 g/L or 10 g/L pepsin. In particular embodiments, the collagen composition is contacted with about 0.5 to 1.0 g/L pepsin in acetic acid solution with pH about 2-3, at about 23°C to 27°C for about 16-24 hours.

The ECM may be contacted with the enzyme in a suitable solution volume:placental tissue weight to remove telopeptide. It is observed that a high volume ratio to placental tissue weight can maximize the effect by pepsin. In certain embodiments, about 1, 2, 4, or 8 volumes of acetic acid solution per placenta is used. In particular embodiments, about 2 volumes of acetic acid solution per placenta is used.

If desired, the ECM can be further processed by fibrillation, e.g., as described in U.S. Pat. Nos. 4,511,653, 4,582,640 and 5,436,135, the contents of which are hereby incorporated by reference in their entireties. If necessary, the collagen composition can be concentrated according to standard techniques prior to fibrillation.

Where desired, the ECM can be cross-linked. In certain embodiments, the ECM is fibrillated prior to cross-linking. The cross-linking can be with any cross-linker known to those of skill in the art, for instance, the cross-linkers described above. In certain embodiments, the cross-linker is glutaraldehyde, and the cross-linking can be carried out according to methods of glutaraldehyde cross-linking of collagen known to those of skill in the art. In other embodiments, the cross-linker is 1,4-butanediol diglycidyl ether or genipin.

In some embodiments, a covalent bond between a cross-linker and a collagen can be reduced, for example to improve stability. The reduction can be accomplished by contacting the ECM, e.g., the collagen in the ECM, with any reducing agent known to those of skill in the art. In certain embodiments, the reducing agent is sodium borohydride, sodium bisulfite, β-mercaptoethanol, mercaptoacetic acid, mercaptoethylamine, benzyl mercaptan, thiocresol, dithiothreitol or a phosphine such as tributylphosphine. In certain embodiments, the collagen in the ECM is cross-linked prior to reduction with the reducing agent. Reduction of collagen compositions and cross-linked collagen compositions is described in U.S. Pat. Nos. 4,185,011, 4,597,762, 5,412,076 and 5,763,579, the contents of which are hereby incorporated by reference in their entirety.

In certain embodiments, the ECM can be further processed by mechanical shearing according to methods known to those of skill in the art. Exemplary shearing techniques are described in U.S. Pat. No. 4,642,117, the contents of which are hereby incorporated by reference in their entirety. In certain embodiments, the ECM is sheared with a tissue homogenizer.

In certain embodiments, steps can be taken to limit native protease activity in the ECM. Suboptimal conditions for proteases may be achieved by formulating the compositions to eliminate or limit the amount of calcium and zinc ions available in solution. Many proteases are active in the presence of calcium and zinc ions and lose much of their activity in calcium and zinc ion free environments. Additives such as metal ion chelators, for example 1,10-phenanthroline and ethylenediaminetetraacetic acid (EDTA), therefore create an environment unfavorable to many proteolytic enzymes. Advantageously, ECM can be prepared selecting conditions of pH, reduced availability of calcium and zinc ions, presence of metal ion chelators and the use of proteolytic inhibitors specific for collagenase. For example, ECM may include a buffered solution of water, pH 5.5 to 8, or pH 7 to 8, free from calcium and zinc ions and including a metal ion chelator such as EDTA. Additionally, control of temperature and time parameters during the treatment of a collagen composition may also be employed to limit the activity of proteases.

### 5.5. Double-Dried Extracellular Matrix Composition

In certain embodiments, the ECM produced, e.g., by any of the methods in the preceding Sections, may be double dried, e.g., by a method comprising, in order: freezing the ECM; lyophilizing the ECM; rehydrating the ECM; and drying the ECM at a temperature of 40°C to 70°C to produce said ECM composition. In certain embodiments, said method does not comprise modification of the ECM with an exogenously introduced protease. In certain embodiments, said drying the ECM is performed at between 50°C to 70°C. Drying of the ECM at room temperature after rehydration (e.g., by application of a vacuum) does not suffice to produce the double-dried ECM provided herein that has the requisite cell attachment and proliferation characteristics.

Said freezing of the ECM can take place at any temperature below 0°C, e.g., at -10°C, -15°C, -20°C, -25°C, -30°C, -35°C, -40°C, -45°C, -50°C, -55°C, -60°C, -65°C, -70°C, -75°C, -80°C, or -85°C, or colder. In certain embodiments, said freezing takes place at a temperature of 0°C to -10°C, -10°C to -20°C, -20°C to -30°C, -30°C to -40°C, -40°C to -50°C, -50°C to -60°C, -60°C to -70°C, or -70°C, to -80°C.

Lyophilization can be accomplished by any means known in the art, and generally proceeds until the ECM composition is substantially dry, e.g., less than about 30%, 25%, 20%, 25%, 20%, 5%, 4%, 3%, 2% or 1% water by weight.

Rehydration of the lyophilized ECM can, for example, be accomplished with any therapeutically or medically-acceptable liquid, buffer, cell culture media, excipient, or the like. In certain embodiments, the liquid used to rehydrate the ECM comprises one or more biomolecules that, e.g., assist cells subsequently added to the ECM to proliferate, assist repair of a tissue in a recipient of the ECM, or both.

After rehydration, and prior to heat drying, the ECM may be formed into any useful shape, e.g., block, sheet, tube (e.g., a closed tube or a tube with a lengthwise slit along part or all of the length of the tube), or other shape. The ECM may then be heat dried to complete formation of the ECM into the desired shape. If the ECM is to be modified or derivatized, in certain embodiments, such modification or derivatization is performed after the double drying procedure is complete.

In certain embodiments, the double dried ECM is prepared as sheets, e.g., 3.5"x3.5" sheets.

### 5.6. Characterization of ECM

### 5.6.1. Biochemical Characterization

Biochemical based assays known in the art and exemplified herein may be used to determine the biochemical compositions of the ECM, either before or after the double-drying method of Section 5.2. Absorbance based assays, e.g., for protein content, include but are not limited to assays that measure absorbance at 280 nm (see, e.g., Layne, E, Spectrophotometric and Turbidimetric Methods for Measuring Proteins, Methods in Enzymology 3: 447-455, (1957); Stoscheck, C M, Quantitation of Protein, Methods in Enzymology 182: 50-69, (1990)), 205 nm, and assays based on the extinction coefficient of the sample (see, e.g., Scopes, R K, Analytical Biochemistry 59: 277, (1974); Stoscheck, C M. Quantitation of Protein, Methods in Enzymology 182: 50-69, (1990)). ECM may be characterized using known assays for, e.g., one or more of collagen type I, collagen type II, collagen type III, collagen type IV, laminin, elastin, fibronectin, and/or glycosaminoglycan.

Colorimetric based assays included but are not limited to modified Lowry assay, biuret assay, Bradford assay, Bicinchoninic Acid (Smith) assay (see, e.g., Stoscheck, C M, Quantitation of Protein, Methods in Enzymology 182: 50-69 (1990)).

In a specific embodiment, measuring the total protein content of ECM comprises use of a Bradford dye-binding assay (Bradford, M., Analytical Biochemistry, 72, 248 (1976), which is incorporated herein by reference in its entirety). A Bradford assay may be carried out, e.g., using the Bradford dye-binding assay available through BIO-RAD, Hercules, Calif., USA. The protein assay is based on the change in color of the dye Coomassie Brilliant Blue R-250 in response to different concentrations of protein. The assay involves developing a standard calibration curve by measuring absorbance (at 595 nanometers) of a series of human collagen standards of known concentrations. The concentration of collagen in an ECM test sample, for example, sample of the amniotic membrane, is determined by referencing to the standard curve. The assay is developed in a standard format that allows measurement of collagen concentration in the range of 0.2-1.4 mg/mL and as a microassay that measures protein concentration up to 25 µg. For the standard assay, ECM dissolved in 100 mM citric acid (pH 2.4) is aliquoted into 1.5 mL microcentrifuge tubes at concentrations of 0.1-1 mg/mL at a total volume of 0.1 mL. To each tube, 1 mL of the Coomassie blue dye is added. Samples are vortexed and allowed to stand at room temperature for 10 minutes. Absorbance is measured at 595 nanometers (nm). For the microassay, ECM dissolved in 100 mM citric acid (pH 2.4) is aliquoted into wells of a 96-well plate at a total volume of 0.1 mL (2.5-30 µg/mL). To each well, 10 µL of dye reagent is added. Samples are vortexed, incubated at room temperature for ten minutes before measuring absorbance in a plate reader at 595 nm. Test samples can be assayed in triplicate. Protein concentrations are determined by referencing to the standard curve. Protein concentration is calculated as a percentage of the total dry weight of the ECM. Within a margin of error of about 10%, the protein content in each of the ECM is essentially 95% or more of the total dry weight of the ECM. Water content may be low and within the experimental error (approximately 10%).

Estimation of the total collagen content of the ECM may be characterized using methods known to one skilled in the art and exemplified herein. In a specific embodiment the collagen content of ECM is measured using a quantitative dye-based assay kit, e.g., the SIRCOL™ kit manufactured by Biocolor Ltd, UK. Collagen types in ECM may be determined using standard methods known in the art and exemplified herein, e.g., ELISA assay, e.g., a sandwich ELISA assay provided, for example, as a kit by Arthrogen-CIA® Collagen-I from Chondrex, Inc., Redmond, Wash., USA. For the Type III and Type IV studies, the primary (Capture Antibody) and secondary antibodies (Detection Antibody) and collagen standards may be obtained from Rockland Immunochemicals, Gilbertsville, Pa. Total elastin content of the ECM may be determined using methods known in the art, using, e.g., a quantitative dye-based assay kit (FASTIN) manufactured by Biocolor Ltd, UK. The assay utilizes 5,10,15,20-tetraphenyl-21,23-porphrine (TPPS) as a specific elastin binding dye (see, e.g., Winkleman, J. (1962), Cancer Research, 22, 589-596). Total glycosaminoglycan (GAG) content of the ECM may be determined using, e.g., a quantitative dye-based assay kit (BLYSCAN) manufactured by Biocolor Ltd, UK. Total laminin content of the ECM may be assayed using methods known in the art, e.g., using a sandwich ELISA assay, e.g., as provided as a kit from Takara Bio Inc., Shiga, Japan (Cat # MKIO7).

Total fibronectin content of the ECM may be assayed using methods known in the art, e.g., using a sandwich ELISA assay provided as a kit from Takara Blo Inc., Shiga, Japan (Cat # MK1 15). Other methods for determining the content of each of the above proteins or biomolecules are well-known in the art. Protocols for determining each are provided, e.g., in U.S. Application Publication No. 2008/0181935, which is hereby incorporated by reference in its entirety.

### 5.6.2. Storage and Handling of ECM

ECM, e.g., in microcarrier form, may be stored at room temperature (e.g., 25°C), or at, e.g., at least 0°C, at least 4°C, at least 10°C, at least 15°C, at least 20°C, at least 25°C, at least 30°C, at least 35°C or at least 40°C, no more than 4°C, no more than 10°C, no more than 15°C, no more than 20°C, no more than 25°C, no more than 30°C, no more than 35°C or no more than 40°C. The ECM may be unrefrigerated, or may be refrigerated at a temperature of about 2 to 8°C. The ECM may be stored under sterile and non-oxidizing conditions. The ECM may be stored in any container suitable for long-term storage. Advantageously, ECM can be stored in a sterile double peel-pouch package.

### 5.6.3. Sterilization

ECM, e.g., in microcarrier form, can be sterilized according to techniques known to those of skill in the art for sterilizing such compositions. The ECM may be sterilized by filtration, e.g., by passage through a filter that allows passage of endotoxins and retains the collagen composition. Any filter of a size, for example 30 kDa, known to those of skill in the art for filtration of endotoxins can be used. The filter may be of a size that retains collagen while allowing endotoxins to pass the filter. In certain embodiments, the filter is between 5 kDa and 100 kDa. In particular embodiments, the filter is about 5 kDa, about 10 kDa, about 15 kDa, about 20 kDa, about 30 kDa, about 40 kDa, about 50 kDa, about 60 kDa, about 70 kDa, about 80 kDa, about 90 kDa or about 100 kDa. The filter can be of any material known to those of skill in the art to be compatible with a collagen composition such as cellulose, polyethersulfone and others apparent to those of skill. The filtration can be repeated as many times as desired by one of skill in the art. Endotoxin can be detected according to standard techniques to monitor clearance.

In certain embodiments, ECM can be filtered to generate ECM free of, or reduced in, viral particles. Advantageously, the filter retains a collagen composition while allowing viral particles to pass through. Any filter known to those of skill in the art to be useful for clearing viruses can be used. For instance, a 1000 kDa filter can be used for clearance, or reduction, of parvovirus, hepatitis A virus and HIV. A 750 kDa filter can be used for clearance, or reduction, of parvovirus and hepatitis A virus. A 500 kDa filter can be used for clearance, or reduction, of parvovirus.

ECM can be prepared which is free of viral particles, or reduced in number of viral particles, by a method comprising the step of contacting the ECM with a filter of a size that allows one or more viral particles to pass through the filter while retaining the ECM. In certain embodiments, the collagen composition is contacted with the filter under conditions that allow one or more viral particles to pass through the filter while retaining a collagen composition. The conditions can be any conditions for filtration known to those of skill in the art, for instance, centrifugation or pumping. The filter should be of a size that retains collagen while allowing one or more viral particles to pass the filter. In certain embodiments, the filter is between 500 kDa and 1000 kDa. In particular embodiments, the filter is about 500 kDa, about 750 kDa or about 1000 kDa. The filter can be of any material known to those of skill in the art to be compatible with a collagen composition such as cellulose, polyethersulfone and others apparent to those of skill. The filtration can be repeated as many times as desired by one of skill in the art. Viral particles can be detected according to standard techniques to monitor filtration.

Sterilization of ECM can also be carried out by electron beam irradiation using methods known to one skilled in the art, e.g., Gorham, D. Byrom (ed.), 1991, Biomaterials, Stockton Press, New York, 55-122. Any dose of radiation sufficient to kill at least 99.9% of bacteria or other potentially contaminating organisms is within the scope of the method. In a particular embodiment, a dose of at least 18-25 kGy is used to achieve the terminal sterilization of ECM.

### 5.7. Formulations of ECM

ECM, as extracted from the placenta, is typically a white paste. In embodiments in which the microcarriers consist essentially of, or consist of, ECM, this paste can be dried, e.g., double-dried, and formed into a shape, e.g., a block, suitable for milling to a powder, wherein the powder comprises pellets or beads of ECM that are of appropriate size for microcarriers.

For applications in which the ECM is used to coat beads or pellets of a second composition (e.g., glass, plastic, DEAE-dextran, or other compositions disclosed elsewhere herein), the ECM can be formulated in water or phosphate buffered saline, e.g., as a solution or suspension, e.g., at 0.1-100 mg/ml, 1-100 mg/ml, 1-75 mg/ml, 1-50 mg/ml, 1-40 mg/ml, 10-40 mg/ml or 20-40 mg/ml ECM. In certain embodiments, the ECM solution or suspension comprises about 5 mg/ml, 10 mg/ml, 15 mg/ml, 20 mg/ml, 25 mg/ml, 30 mg/ml, 35 mg/ml, 40 mg/ml, 45 mg/ml or 50 mg/ml collagen.

The ECM microcarriers can be formulated to release an active ingredient, e.g., a biomolecule, during culture of cells. For example, the microcarriers may be impregnated, either during production or during preparation for surgery, with a biomolecule, e.g., an antibiotic (such as Clindamycin, Minocycline, Doxycycline, Gentamycin), a hormone, a growth factor, an antitumor agent, an anti-fungal agent, an anti-viral agent, an anti-histamine, an anti-inflammatory agent, a wound healing agents (such as cytokines including but not limited to PDGF, TGF; thymosin), and the like.

In yet other embodiments, double-dried ECM may be combined with a hydrogel. Any hydrogel known to one skilled in the art may be used, e.g., any of the hydrogel compositions disclosed in Graham, 1998, Med. Device Technol. 9(1): 18-22; Peppas et al., 2000, Eur. J. Pharm. Biopharm. 50(1): 27-46; Nguyen et al., 2002, Biomaterials, 23(22): 4307-14; Henincl et al., 2002, Adv. Drug Deliv. Rev 54(1): 13-36; Skelhorne et al., 2002, Med. Device. Technol. 13(9): 19-23; or Schmedlen et al., 2002, Biomaterials 23: 4325-32. In a specific embodiment, the hydrogel is applied onto the ECM, i.e., discharged on the surface of the collagen composition. The hydrogel for example, may be sprayed onto the double-dried ECM, saturated on the surface of the double-dried ECM, soaked with the double-dried ECM, bathed with the double-dried ECM or coated onto the surface of the double-dried ECM. The hydrogels useful in the methods and compositions provided herein can be made from any water-interactive, or water soluble polymer known in the art, including but not limited to, polyvinylalcohol (PVA), polyhydroxyethyl methacrylate, polyethylene glycol, polyvinyl pyrrolidone, hyaluronic acid, dextran or derivatives and analogs thereof.

### 5.8. Placental Stem Cells

The microcarriers comprising ECM can be used to culture attachment-dependent cells e.g., adult cells or stem cells, wherein the cells adhere to ECM. Preferably, the cells are placental stem cells such as those described in U.S. Patent Nos. 7,045,148; 7,468,276; 8,057,788 and 8,202,703, the disclosures of which are hereby incorporated by reference in their entireties.

The number of stem or progenitor cells cultured using the ECM-containing microcarriers can, in various embodiments include at least 1 X 10⁶, 3 X 10⁶, 1 X 10⁷, 3 X 10⁷, 1 X 10⁸, 3 X 10⁸, 1 X 10⁹, 3 X 10⁹, 1 X 10¹⁰, 3 X 10¹⁰, 1 X 10¹¹, 3 X 10¹¹, or 1 X 10¹²; or may be no more than 1 X 10⁶, 3 X 10⁶, 1 X 10⁷, 3 X 10⁷, 1 X 10⁸, 3 X 10⁸, 1 X 10⁹, 3 X 10⁹, 1 X 10¹⁰, 3 X 10¹⁰, 1 X 10¹¹, 3 X 10¹¹, or 1 X 10¹² stem or progenitor cells.

In certain embodiments, the cells being cultured using microcarriers comprising ECM are CD34- placental stem cells. CD34- placental stem cells are stem cells, obtainable from placental tissue, that adhere to a tissue culture substrate and have the capacity to differentiate into nonplacental cell types. Placental stem cells can be either fetal or maternal in origin (that is, can have the genotype of either the mother or fetus). Populations of placental stem cells, or populations of cells comprising placental stem cells, can comprise placental stem cells that are solely fetal or maternal in origin, or can comprise a mixed population of placental stem cells of both fetal and maternal origin. The placental stem cells, and populations of cells comprising the placental stem cells, can be identified and selected by the morphological, marker, and culture characteristic discussed below.

The placental stem cells generally express the markers CD10, CD73, CD105, CD200, and/or OCT-4, and do not express HLA-G+, CD34, CD38, or CD45. Placental stem cells can also express HLA-ABC (MHC-1) and HLA-DR. Thus, in one embodiment, the stem cells that can be combined with the ECM are CD200+ or HLA-G-. In another embodiment, the placental stem cells are CD73+, CD105+, and CD200+. In another embodiment, the placental stem cells are CD200+ and OCT-4+. In another embodiment, the placental stem cells are CD73+, CD105+ and HLA-G-.

In certain embodiments, the isolated placental stem cells are CD34-, CD10+ and CD105+ as detectable by flow cytometry. In another specific embodiment, the isolated CD34-, CD10+, CD105+ placental cells have the potential to differentiate into cells of a neural phenotype, cells of an osteogenic phenotype, and/or cells of a chondrogenic phenotype. In another specific embodiment, the isolated CD34-, CD10+, CD105+ placental cells are additionally CD200+. In another specific embodiment, the isolated CD34-, CD10+. CD105+ placental cells are additionally CD45- or CD90+. In another specific embodiment, the isolated CD34-, CD10+, CD105+ placental cells are additionally CD45- and CD90+, as detectable by flow cytometry. In another specific embodiment, the isolated CD34-, CD10+, CD105+, CD200+ placental cells are additionally CD90+ or CD45-, as detectable by flow cytometry. In another specific embodiment, the isolated CD34-, CD10+, CD105+, CD200+ placental cells are additionally CD90+ and CD45-, as detectable by flow cytometry, i.e., the cells are CD34-, CD10+, CD45-, CD90+, CD105+ and CD200+. In another specific embodiment, said CD34-, CD10+, CD45-, CD90+, CD105+, CD200+ cells are additionally CD80- and CD86-.

In certain embodiments, said placental stem cells are CD34-, CD10+, CD105+ and CD200+, and one or more of CD38-, CD45-, CD80-, CD86-, CD133-, HLA-DR,DP,DQ-, SSEA3-, SSEA4-, CD29+, CD44+, CD73+, CD90+, CD105+, HLA-A,B,C+, PDL1+, ABC-p+, and/or OCT-4+, as detectable by flow cytometry. In other embodiments, any of the CD34-, CD10+, CD105+ cells described above are additionally one or more of CD29+, CD38-, CD44+, CD54+, SH3+ or SH4+. In another specific embodiment, the cells are additionally CD44+. In another specific embodiment of any of the isolated CD34-, CD10+, CD105+ placental cells above, the cells are additionally one or more of CD117-, CD133-, KDR- (VEGFR2-), HLA-A,B,C+, HLA-DP,DQ,DR-, or Programmed Death-1 Ligand (PDL1) +, or any combination thereof.

In another embodiment, the CD34-, CD10+, CD105+ placental stem cells are additionally one or more of CD13+, CD29+, CD33+, CD38-, CD44+, CD45-, CD54+, CD62E-, CD62L-, CD62P-, SH3+ (CD73+), SH4+ (CD73+), CD80-, CD86-, CD90+, SH2+ (CD105+), CD106/VCAM+, CD117-, CD144/VE-cadherin_{low}, CD184/CXCR4-, CD200+, CD133-, OCT-4+, SSEA3-, SSEA4-, ABC-p+, KDR- (VEGFR2-), HLA-A,B,C+, HLA-DP,DQ,DR-, HLA-G-, or Programmed Death-1 Ligand (PDL1)+, or any combination thereof. In another embodiment, the CD34-, CD10+, CD105+ cells are additionally CD13+, CD29+, CD33+, CD38-, CD44+, CD45-, CD54/ICAM+, CD62E-, CD62L-, CD62P-, SH3+ (CD73+), SH4+ (CD73+), CD80-, CD86-, CD90+, SH2+ (CD105+), CD106/VCAM+, CD117-, CD144/VE-cadherin^{low}, CD184/CXCR4-, CD200+, CD133-, OCT-4+, SSEA3-, SSEA4-, ABC-p+, KDR-(VEGFR2-), HLA-A,B,C+, HLA-DP,DQ,DR-, HLA-G-, and Programmed Death-1 Ligand (PDL1)+.

In another specific embodiment, any of the placental stem cells described herein are additionally ABC-p+, as detectable by flow cytometry, or OCT-4+ (POU5F1), as determined by reverse-transcriptase polymerase chain reaction (RT-PCR), wherein ABC-p is a placenta-specific ABC transporter protein (also known as breast cancer resistance protein (BCRP) and as mitoxantrone resistance protein (MXR)), and OCT-4 is the Octamer-4 protein (POU5F1). In another specific embodiment, any of the placental stem cells described herein are additionally SSEA3- or SSEA4-, as determined by flow cytometry, wherein SSEA3 is Stage Specific Embryonic Antigen 3, and SSEA4 is Stage Specific Embryonic Antigen 4. In another specific embodiment, any of the placental stem cells described herein are additionally SSEA3- and SSEA4-.

In another specific embodiment, any of the placental cells described herein are additionally one or more of MHC-I+ (e.g., HLA-A,B,C+), MHC-II- (e.g., HLA-DP,DQ,DR-) or HLA-G-. In another specific embodiment, any of the placental stem cells described herein are additionally one or more of MHC-I+ (e.g., HLA-A,B,C+), MHC-IV (e.g., HLA-DP,DQ,DR-) and HLA-G-.

Also provided herein are populations of the isolated placental stem cells, or populations of cells, e.g., populations of placental cells, comprising, e.g., that are enriched for, the isolated placental stem cells, that are useful in the methods and compositions disclosed herein. Preferred populations of cells comprising the isolated placental stem cells, wherein the populations of cells comprise, e.g., at least 10%, 15%, 20%, 25%, 30%, 35%, 40%, 45%, 50%, 55%, 60%, 65%, 70%, 75%, 80%, 85%, 90%, 95% or 98% isolated placental stem cells that are CD10+, CD105+ and CD34-; that is, at least 10%, 15%, 20%, 25%, 30%, 35%, 40%, 45%, 50%, 55%, 60%, 65%, 70%, 75%, 80%, 85%, 90%, 95% or 98% of cells in said population are isolated placental stem cells that are CD10+, CD105+ and CD34-. In a specific embodiment, the isolated CD34-, CD10+, CD105+ placental stem cells are additionally CD200+. In another specific embodiment, the isolated CD34-, CD10+, CD105+, CD200+ placental stem cells are additionally CD90+ or CD45-, as detectable by flow cytometry. In another specific embodiment, the isolated CD34-, CD10+, CD105+, CD200+ placental stem cells are additionally CD90+ and CD45-, as detectable by flow cytometry. In another specific embodiment, any of the isolated CD34-, CD10+, CD105+ placental stem cells described above are additionally one or more of CD29+, CD38-, CD44+, CD54+, SH3+ or SH4+. In another specific embodiment, the isolated CD34-, CD10+, CD105+ placental stem cells, or isolated CD34-, CD10+, CD105+, CD200+ placental stem cells, are additionally CD44+. In a specific embodiment of any of the populations of cells comprising isolated CD34-, CD10+, CD105+ placental stem cells above, the isolated placental stem cells are additionally one or more of CD13+, CD29+, CD33+, CD38-, CD44+, CD45-, CD54-, CD62E-, CD62L-, CD62P-, SH3+ (CD73+), SH4+ (CD73+), CD80-, CD86-, CD90+, SH2+ (CD105+), CD106/VCAM+, CD117-, CD144NE-cadherin^{low}, CD184/CXCR4-, CD200+, CD133-, OCT-4+, SSEA3-, SSEA4-, ABC-p+, KDR- (VEGFR2-), HLA-A,B,C+, HLA-DP,DQ,DR-, HLA-G-, or Programmed Death-1 Ligand (PDL1) +, or any combination thereof. In another specific embodiment, the CD34-, CD10+, CD105+ placental stem cells are additionally CD13+, CD29+, CD33+, CD38-, CD44+, CD45-, CD54/ICAM+, CD62E-, CD62L-, CD62P-, SH3+ (CD73+), SH4+ (CD73+), CD80-, CD86-, CD90+, SH2+ (CD105+), CD106/VCAM+, CD117-, CD144NE-cadherin^{low}, CD184/CXCR4-, CD200+, CD133-, OCT-4+, SSEA3-, SSEA4-, ABC-p+, KDR- (VEGFR2-), HLA-A,B,C+, HLA-DP,DQ,DR-, HLA-G-, and Programmed Death-1 Ligand (PDL1)+.

In certain embodiments, the isolated placental stem cells useful in the methods and compositions described herein are one or more, or all, of CD10+, CD29+, CD34-, CD38-, CD44+, CD45-, CD54+, CD90+, SH2+, SH3+, SH4+, SSEA3-, SSEA4-, OCT-4+, and ABC-p+, wherein said isolated placental stem cells are obtained by physical and/or enzymatic disruption of placental tissue. In a specific embodiment, the isolated placental stem cells are OCT-4+ and ABC-p+. In another specific embodiment, the isolated placental stem cells are OCT-4+ and CD34-, wherein said isolated placental stem cells have at least one of the following characteristics: CD10+, CD29+, CD44+, CD45-, CD54+, CD90+, SH3+, SH4+, SSEA3-, and SSEA4-. In another specific embodiment, the isolated placental stem cells are OCT-4+, CD34-, CD10+, CD29+, CD44+, CD45-, CD54+, CD90+, SH3+, SH4+, SSEA3-, and SSEA4-. In another embodiment, the isolated placental stem cells are OCT-4+, CD34-, SSEA3-, and SSEA4-. In another specific embodiment, the isolated placental stem cells are OCT-4+ and CD34-, and either SH2+ or SH3+. In another specific embodiment, the isolated placental stem cells are OCT-4+, CD34-, SH2+, and SH3+. In another specific embodiment, the isolated placental stem cells are OCT-4+, CD34-, SSEA3-, and SSEA4-, and are either SH2+ or SH3+. In another specific embodiment, the isolated placental stem cells are OCT-4+ and CD34-, and either SH2+ or SH3+, and are at least one of CD10+, CD29+, CD44+, CD45-, CD54+, CD90+, SSEA3-, or SSEA4-. In another specific embodiment, the isolated placental stem cells are OCT-4+, CD34-, CD10+, CD29+, CD44+, CD45-, CD54+, CD90+, SSEA3-, and SSEA4-, and either SH2+ or SH3+.

In another embodiment, the isolated placental stem cells are SH2+, SH3+, SH4+ and OCT-4+. In another specific embodiment, the isolated placental stem cells are CD10+, CD29+, CD44+, CD54+, CD90+, CD34-, CD45-, SSEA3-, or SSEA4-. In another embodiment, the isolated placental stem cells are SH2+, SH3+, and SH4+. SSEA3- and SSEA4-. In another specific embodiment, the isolated placental stem cells are SH2+, SH3+, SH4+, SSEA3- and SSEA4-, CD10+, CD29+, CD44+, CD54+, CD90+, OCT-4+, CD34- or CD45-.

In another embodiment, the isolated placental stem cells useful in the methods and compositions disclosed herein are CD10+, CD29+, CD34-, CD44+, CD45-, CD54+, CD90+, SH2+, SH3+, and SH4+; wherein said isolated placental stem cells are additionally one or more of OCT-4+, SSEA3- or SSEA4-.

In certain embodiments, isolated placental stem cells are CD200+ or HLA-G-. In a specific embodiment, the isolated placental stem cells are CD200+ and HLA-G-. In another specific embodiment, the isolated placental stem cells are additionally CD73+ and CD105+. In another specific embodiment, the isolated placental stem cells are additionally CD34-, CD38- or CD45-. In another specific embodiment, the isolated placental stem cells are additionally CD34-, CD38- and CD45-. In another specific embodiment, said placental stem cells are CD34-, CD38-, CD45-, CD73+ and CD105+.

In another embodiment, the isolated placental stem cells are CD73+, CD105+, and CD200+. In another specific embodiment, the isolated placental stem cells are HLA-G-. In another specific embodiment, the isolated placental stem cells are CD34-, CD38- or CD45-. In another specific embodiment, the isolated placental stem cells are CD34-, CD38- and CD45-. In another specific embodiment, the isolated placental stem cells are CD34-, CD38-, CD45-, and HLA-G-.

In certain other embodiments, the isolated placental stem cells are one or more of CD10+, CD29+, CD34-, CD38-, CD44+, CD45-, CD54+, CD90+, SH2+, SH4+, SSEA3-, SSEA4-, OCT-4+, HLA-G- or ABC-p+. In a specific embodiment, the isolated placental stem cells are CD10+, CD29+. CD34-, CD38-, CD44+, CD45-, CD54+, CD90+, SH2+, SH3+, SH4+, SSEA3-, SSEA4-, and OCT-4+. In another specific embodiment, the isolated placental stem cells are CD10+, CD29+, CD34-, CD38-, CD45-, CD54+, SH2+, SH3+, and SH4+. In another specific embodiment, the isolated placental stem cells are CD10+, CD29+, CD34-, CD38-, CD45-, CD54+, SH2+, SH3+, SH4+ and OCT-4+. In another specific embodiment, the isolated placental stem cells are CD10+, CD29+, CD34-, CD38-, CD44+, CD45-, CD54+, CD90+, HLA-G-, SH2+, SH3+, and SH4+. In another specific embodiment, the isolated placental stem cells are OCT-4+ and ABC-p+. In another specific embodiment, the isolated placental stem cells are SH2+, SH3+, SH4+ and OCT-4+. In another embodiment, the isolated placental stem cells are OCT-4+, CD34-, SSEA3-, and SSEA4-. In a specific embodiment, said isolated OCT-4+, CD34-, SSEA3-, and SSEA4-placental cells are additionally CD10+, CD29+, CD34-, CD44+, CD45-, CD54+, CD90+, SH2+, SH3+, and SH4+. In another embodiment, the isolated placental stem cells are OCT-4+ and CD34-, and either SH3+ or SH4+. In another embodiment, the isolated placental stem cells are CD34- and either CD10+, CD29+, CD44+, CD54+, CD90+, or OCT-4+.

In another embodiment, the isolated placental stem cells are CD200+ and OCT-4+. In a specific embodiment, the isolated placental stem cells are CD73+ and CD105+. In another specific embodiment, said isolated placental stem cells are HLA-G-. In another specific embodiment, said isolated CD200+, OCT-4+ placental stem cells are CD34-, CD38- or CD45-. In another specific embodiment, said isolated CD200+, OCT-4+ placental stem cells are CD34-, CD38- and CD45-. In another specific embodiment, said isolated CD200+, OCT-4+ placental stem cells are CD34-, CD38-, CD45-, CD73+, CD105+ and HLA-G-.

In another embodiment, the isolated placental stem cells useful in the methods and compositions described herein are CD73+, CD105+ and HLA-G-. In another specific embodiment, the isolated CD73+, CD105+ and HLA-G- placental stem cells are additionally CD34-, CD38- or CD45-. In another specific embodiment, the isolated CD73+, CD105+, HLA-G- placental cells are additionally CD34-, CD38- and CD45-. In another specific embodiment, the isolated CD73+, CD105+, HLA-G- placental stem cells are additionally OCT-4+.

In another specific embodiment, the isolated CD73+, CD105+, HLA-G- placental stem cells are additionally CD200+. In another specific embodiment, the isolated CD73+, CD105+, HLA-G- placental stem cells are additionally CD34-, CD38-, CD45-, OCT-4+ and CD200+. In another specific embodiment, the isolated CD73+, CD105+, HLA-G- placental stem cells facilitate the formation of embryoid-like bodies in a population of placental cells comprising said cells, when the population is cultured under conditions that allow the formation of embryoid-like bodies. In another specific embodiment, said the isolated CD73+, CD105+, HLA-G- placental stem cells are isolated away from placental cells that are not the isolated CD73+, CD105+, HLA-G- placental stem cells. In another specific embodiment, said the isolated CD73+, CD105+, HLA-G- placental stem cells are isolated away from placental cells that do not display these markers.

In another embodiment, the isolated placental stem cells useful in the methods and compositions described herein are CD73+ and CD105+ and facilitate the formation of one or more embryoid-like bodies in a population of isolated placental cells comprising said CD73+, CD105+ placental stem cells when said population is cultured under conditions that allow formation of embryoid-like bodies. In another specific embodiment, said isolated CD73+, CD105+ placental stem cells are additionally CD34-, CD38- or CD45-. In another specific embodiment, said isolated CD73+, CD105+ placental stem cells are additionally CD34-, CD38-and CD45-. In another specific embodiment, said isolated CD73+, CD105+ placental stem cells are additionally OCT-4+. In another specific embodiment, said isolated CD73+, CD105+ placental stem cells are additionally OCT-4+, CD34-, CD38- and CD45-. In another specific embodiment, said isolated CD73+, CD105+ placental cells are isolated away from placental cells that are not said cells. In another specific embodiment, said isolated CD73+, CD105+ placental cells are isolated away from placental cells that do not display these characteristics.

In another embodiment, the isolated placental stem cells HLA-A,B,C+, CD45-, CD133-and CD34-. In another embodiment, a cell population culturable using the ECM-containing microcarriers is a population of cells comprising isolated placental stem cells, wherein at least about 70%, at least about 80%, at least about 90%, at least about 95% or at least about 99% of cells in said population of cells are isolated HLA-A,B,C+, CD45-, CD133- and CD34- placental stem cells. In a specific embodiment, said isolated placental stem cell or population of isolated placental stem cells is isolated away from placental cells that are not HLA-A,B,C+, CD45-, CD133- and CD34- placental stem cells. In another specific embodiment, said isolated placental stem cells are non-maternal in origin. In another specific embodiment, said population of isolated placental stem cells are substantially free of maternal components; e.g., at least about 40%, 45%, 5-0%, 55%, 60%, 65%, 70%, 75%, 90%, 85%, 90%, 95%, 98% or 99% of said cells in said population of isolated placental cells are non-maternal in origin.

In another embodiment, the isolated placental stem cells are CD10+, CD13+, CD33+, CD45-, CD117- and CD133-. In another embodiment, a cell population culturable using the ECM-containing microcarriers is a population of cells comprising isolated placental stem cells, wherein at least about 70%, at least about 80%, at least about 90%, at least about 95% or at least about 99% of cells in said population of cells are isolated CD10+, CD13+, CD33+, CD45-, CD117- and CD133- placental stem cells. In a specific embodiment, said isolated placental stem cells or population of isolated placental stem cells is isolated away from placental cells that are not said isolated placental stem cells. In another specific embodiment, said isolated CD10+, CD13+, CD33+, CD45-, CD117- and CD133- placental cells are non-maternal in origin, i.e., have the fetal genotype. In another specific embodiment, at least about 40%, 45%, 50%, 55%, 60%, 65%, 70%, 75%, 90%, 85%, 90%, 95%, 98% or 99% of said cells in said population of isolated placental stem cells, are non-maternal in origin. In another specific embodiment, said isolated placental stem cells or population of isolated placental stem cells are isolated away from placental cells that do not display these characteristics.

In another embodiment, the isolated placental stem cells are CD10+ CD33-, CD44+, CD45-, and CD117-. In another embodiment, a cell population culturable using the ECM-containing microcarriers is a population of cells comprising, e.g., enriched for, isolated placental stem cells, wherein at least about 70%, at least about 80%, at least about 90%, at least about 95% or at least about 99% of cells in said population of cells are isolated CD10+ CD33-, CD44+, CD45-, and CD117- placental stem cells. In a specific embodiment, said isolated placental cell or population of isolated placental stem cells is isolated away from placental cells that are not said cells. In another specific embodiment, said isolated placental stem cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated placental stem cell or population of isolated placental stem cells is isolated away from placental cells that do not display these markers.

In another embodiment, the isolated placental stem cells are CD10+ CD13-, CD33-, CD45-, and CD117-. In another embodiment, a cell population culturable using the ECM-containing microcarriers is a population of cells comprising, e.g., enriched for, isolated CD10+, CD13-, CD33-, CD45-, and CD117- placental stem cells, wherein at least about 70%, at least about 80%, at least about 90%, at least about 95% or at least about 99% of cells in said population are CD10+ CD13-, CD33-, CD45-, and CD117- placental cells. In a specific embodiment, said isolated placental stem cells or population of isolated placental stem cells are isolated away from placental cells that are not said cells. In another specific embodiment, said isolated placental stem cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated placental stem cell or population of isolated placental stem cells is isolated away from placental cells that do not display these characteristics.

In another embodiment, the isolated placental stem cells are HLA A,B,C+, CD45-, CD34-, and CD133-, and are additionally CD10+, CD13+, CD38+, CD44+, CD90+, CD105+, CD200+ and/or HLA-G-, and/or negative for CD117. In another embodiment, a cell population culturable using the ECM-containing microcarriers is a population of cells comprising isolated placental stem cells, wherein at least about 20%, 25%, 30%, 35%, 40%, 45%, 50%, 55%, 60%, 65%, 70%, 75%, 80%, 85%, 90%, 95%, 98% or about 99% of the cells in said population are isolated placental stem cells that are HLA A,B,C+, CD45-, CD34-, CD133-, and that are additionally positive for CD10, CD13, CD38, CD44, CD90, CD105, CD200, and/or negative for CD117 and/or HLA-G. In a specific embodiment, said isolated placental stem cells or population of isolated placental stem cells are isolated away from placental cells that are not said cells. In another specific embodiment, said isolated placental stem cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated placental stem cells or population of isolated placental stem cells are isolated away from placental cells that do not display these markers.

In another embodiment, the isolated placental stem cells are CD200+ and CD10+, as determinable by antibody binding, and CD117-, as determinable by either antibody binding or RT-PCR. In another embodiment, the isolated placental stem cells are CD10+, CD29+, CD54+, CD200+, HLA-G-, MHC class I+ and β-2-microglobulin+. In another embodiment, the isolated placental stem cells are placental cells wherein the expression of at least one cellular marker is at least two-fold higher than for a mesenchymal stem cell (e.g., a bone marrow-derived mesenchymal stem cell). In another specific embodiment, said isolated placental stem cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin.

In another embodiment, the isolated placental stem cells are isolated placental stem cells that are one or more of CD10+, CD29+, CD44+, CD45-, CD54/ICAM+, CD62E-. CD62L-, CD62P-, CD80-, CD86-, CD103-, CD104-, CD105+, CD106/VCAM+, CD144/VE-cadherin^{low}, CD184/CXCR4-, β-microglobulin^{low}, MHC-I^{low}, MHC-II-, HLA-G^{low}, and/or PDL1^{low}. In a specific embodiment, the isolated placental stem cells are at least CD29+ and CD54+. In another specific embodiment, the isolated placental stem cells are at least CD44+ and CD106+. In another specific embodiment, the isolated placental stem cells are at least CD29+.

In another embodiment, the isolated placental stem cells are isolated placental stem cells that are one or more, or all, of CD10+, CD29+, CD34-, CD38-, CD44+, CD45-, CD54+, CD90+, SH2+, SH3+, SH4+, SSEA3-, SSEA4-, OCT-4+, and ABC-p+, where ABC-p is a placenta-specific ABC transporter protein (also known as breast cancer resistance protein (BCRP) and as mitoxantrone resistance protein (MXR)), wherein said isolated placental stem cells are obtained, e.g., by perfusion of a mammalian, e.g., human, placenta that has been drained of cord blood and perfused to remove residual blood.

In another specific embodiment of any of the above characteristics, expression of the cellular marker (e.g., cluster of differentiation or immunogenic marker) is determinable by flow cytometry; in another specific embodiment, expression of the marker is determinable by RT-PCR.

Gene profiling confirms that isolated placental stem cells, and populations of isolated placental stem cells, are distinguishable from other cells, e.g., mesenchymal stem cells, e.g., bone marrow-derived mesenchymal stem cells. The isolated placental stem cells described herein can be distinguished from, e.g., mesenchymal stem cells on the basis of the expression of one or more genes, the expression of which is significantly higher in the isolated placental stem cells in comparison to bone marrow-derived mesenchymal stem cells. In particular, the isolated placental stem cells, useful in the methods of treatment provided herein, can be distinguished from mesenchymal stem cells on the basis of the expression of one or more genes, the expression of which is significantly higher (that is, at least twofold higher) in the isolated placental stem cells than in an equivalent number of bone marrow-derived mesenchymal stem cells, wherein the one or more genes are ACTG2, ADARB1, AMIGO2, ARTS-1, B4GALT6, BCHE, C11orf9, CD200, COL4A1, COL4A2, CPA4, DMD, DSC3, DSG2, ELOVL2, F2RL1, FLJ10781, GATA6, GPR126, GPRC5B, ICAM1, IER3, IGFBP7, ILIA, IL6, IL18, KRT18, KRT8, LIPG, LRAP, MATN2, MEST, NFE2L3, NUAK1, PCDH7, PDLIM3, PKP2, RTN1, SERPINB9, ST3GAL6, ST6GALNAC5, SLC12A8, TCF21, TGFB2, VTN, ZC3H12A, or a combination of any of the foregoing, when the cells are grown under equivalent conditions. See, e.g., U.S. Patent Application Publication No. 2007/0275362, the disclosure of which is incorporated herein by reference in its entirety. In certain specific embodiments, said expression of said one or more genes is determinable, e.g., by RT-PCR or microarray analysis, e.g., using a U133-A microarray (Affymetrix). In another specific embodiment, said isolated placental stem cells express said one or more genes when cultured for a number of population doublings, e.g., anywhere from about 3 to about 35 population doublings, in a medium comprising DMEM-LG (e.g., from Gibco); 2% fetal calf serum (e.g., from Hyclone Labs.); IX insulin-transferrin-selenium (ITS); IX linoleic acid-bovine serum albumin (LA-BSA); 10⁻⁹ M dexamethasone (e.g., from Sigma); 10⁻⁴ M ascorbic acid 2-phosphate (e.g., from Sigma); epidermal growth factor 10 ng/mL (e.g., from R&D Systems); and platelet-derived growth factor (PDGF-BB) 10 ng/mL (e.g., from R&D Systems). In another specific embodiment, the isolated placental cell-specific gene is CD200.

Specific sequences for these genes can be found in GenBank at accession nos. NM_001615 (ACTG2), BC065545 (ADARB1), (NM_181847 (AMIGO2), AY358590 (ARTS-1), BC074884 (B4GALT6), BC008396 (BCHE), BCO20196 (C110rf9), BCO31103 (CD200), NM_001845 (COL4A1), NM_001846 (COL4A2), BCO52289 (CPA4), BC094758 (DMD), AF293359 (DSC3), NM_001943 (DSG2), AF338241 (ELOVL2), AY336105 (F2RL1), NM_018215 (FLJ10781), AY416799 (GATA6), BC075798 (GPR126), NM_016235 (GPRC5B), AF340038 (ICAM1), BC000844 (IER3), BC066339 (IGFBP7), BC013142 (ILIA), BT019749 (IL6), BC007461 (IL18), (BC072017) KRT18, BC075839 (KRT8), BC060825 (LIPG), BC065240 (LRAP), BC010444 (MATN2), BC011908 (MEST), BC068455 (NFE2L3), NM_014840 (NUAK1), AB006755 (PCDH7), NM_014476 (PDLIM3), BC126199 (PKP-2), BC090862 (RTN1), BC002538 (SERPINB9), BCO23312 (ST3GAL6), BC001201 (ST6GALNAC5), BC126160 or BC065328 (SLC12A8), BCO25697 (TCF21), BC096235 (TGFB2), BC005046 (VTN), and BC005001 (ZC3H12A) as of March 2008.

In certain specific embodiments, said isolated placental stem cells express each of ACTG2, ADARB1, AMIGO2, ARTS-1, B4GALT6, BCHE, C11orf9, CD200, COL4A1, COL4A2, CPA4, DMD, DSC3, DSG2, ELOVL2, F2RL1, FLJ10781, GATA6, GPR126, GPRC5B, ICAM1, IER3, IGFBP7, ILIA, IL6, IL18, KRT18, KRT8, LIPG, LRAP, MATN2, MEST, NFE2L3, NUAK1, PCDH7, PDLIM3, PKP2, RTN1, SERPINB9, ST3GAL6, ST6GALNAC5, SLC12A8, TCF21, TGFB2, VTN, and ZC3H12A at a detectably higher level than an equivalent number of bone marrow-derived mesenchymal stem cells, when the cells are grown under equivalent conditions.

In specific embodiments, the placental cells express CD200 and ARTS1 (aminopeptidase regulator of type 1 tumor necrosis factor); ARTS-1 and LRAP (leukocyte-derived arginine aminopeptidase); IL6 (interleukin-6) and TGFB2 (transforming growth factor, beta 2); IL6 and KRT18 (keratin 18); IER3 (immediate early response 3), MEST (mesoderm specific transcript homolog) and TGFB2; CD200 and IER3; CD200 and IL6; CD200 and KRT18; CD200 and LRAP; CD200 and MEST; CD200 and NFE2L3 (nuclear factor (erythroid-derived 2)-like 3); or CD200 and TGFB2 at a detectably higher level than an equivalent number of bone marrow-derived mesenchymal stem cells (BM-MSCs) wherein said bone marrow-derived mesenchymal stem cells have undergone a number of passages in culture equivalent to the number of passages said isolated placental stem cells have undergone. In other specific embodiments, the placental cells express ARTS-1, CD200, IL6 and LRAP; ARTS-1, IL6, TGFB2, IER3, KRT18 and MEST; CD200, IER3, IL6, KRT18, LRAP, MEST, NFE2L3, and TGFB2; ARTS-1, CD200, IER3, IL6, KRT18, LRAP, MEST, NFE2L3, and TGFB2; or IER3, MEST and TGFB2 at a detectably higher level than an equivalent number of bone marrow-derived mesenchymal stem cells BM-MSCs, wherein said bone marrow-derived mesenchymal stem cells have undergone a number of passages in culture equivalent to the number of passages said isolated placental stem cells have undergone.

Expression of the above-referenced genes can be assessed by standard techniques. For example, probes based on the sequence of the gene(s) can be individually selected and constructed by conventional techniques. Expression of the genes can be assessed, e.g., on a microarray comprising probes to one or more of the genes, e.g. an Affymetrix GENECHIP™ Human Genome U133A 2.0 array, or an Affymetrix GENECHIP™ Human Genome U133 Plus 2.0 (Santa Clara, Calif.). Expression of these genes can be assessed even if the sequence for a particular GenBank accession number is amended because probes specific for the amended sequence can readily be generated using well-known standard techniques.

The level of expression of these genes can be used to confirm the identity of a population of isolated placental stem cells, to identify a population of cells as comprising at least a plurality of isolated placental stem cells, or the like. Populations of isolated placental stem cells, the identity of which is confirmed, can be clonal, e.g., populations of isolated placental stem cells expanded from a single isolated placental cell, or a mixed population of stem cells, e.g., a population of cells comprising solely isolated placental stem cells that are expanded from multiple isolated placental stem cells, or a population of cells comprising isolated placental stem cells, as described herein, and at least one other type of cell.

The placental stem cells can be obtained by perfusion, e.g., produced according to a method comprising perfusing a mammalian placenta that has been drained of cord blood and perfused to remove residual blood; perfusing said placenta with a perfusion solution; and collecting said perfusion solution, wherein said perfusion solution after perfusion comprises a population of placental cells that comprises placental stem cells; and isolating a plurality of said placental stem cells from said population of cells. In a specific embodiment, the perfusion solution is passed through both the umbilical vein and umbilical arteries and collected after it exudes from the placenta. Populations of placental stem cells produced by this method typically comprise a mixture of fetal and maternal cells. In another specific embodiment, the perfusion solution is passed through the umbilical vein and collected from the umbilical arteries, or passed through the umbilical arteries and collected from the umbilical vein. Populations of placental stem cells produced by this method typically are substantially exclusively fetal in origin; that is, e.g., greater than 90%, 95%, 99%, or 99.5% of the placental stem cells in the population are fetal in origin.

In various embodiments, the placental stem cells, contained within a population of cells obtained from perfusion of a placenta, are at least 50%, 60%, 70%, 80%, 90%, 95%, 99% or at least 99.5% of said population of placental cells. In another specific embodiment, the placental stem cells collected by perfusion comprise fetal and maternal cells. In another specific embodiment, the placental stem cells collected by perfusion are at least 50%, 60%, 70%, 80%, 90%, 95%, 99% or at least 99.5% fetal cells.

Placental stem cells can also be isolated from a mammalian placenta by physical disruption, e.g., enzymatic digestion, of the organ or a portion thereof. For example, the placenta, or a portion thereof, may be, e.g., crushed, sheared, minced, diced, chopped, macerated or the like, and the tissue subsequently digested with one or more enzymes. The placenta, or a portion thereof, may also be physically disrupted and digested with one or more enzymes, and the resulting material then immersed in, or mixed into, e.g., medium or buffer. Any method of physical disruption can be used, provided that the method of disruption leaves a plurality, more preferably a majority, and more preferably at least 60%, 70%, 80%, 90%, 95%, 98%, or 99% of the cells in said organ viable, as determined by, e.g., trypan blue exclusion.

The placenta can be dissected into components prior to physical disruption and/or enzymatic digestion and stem cell recovery. For example, placental stem cells can be obtained from the amniotic membrane, chorion, umbilical cord, placental cotyledons, or any combination thereof. Typically, placental stem cells can be obtained by disruption of a small block of placental tissue, e.g., a block of placental tissue that is about 1, 2, 3, 4, 5, 6, 7, 8, 9, 10, 20, 30, 40, 50, 60, 70, 80, 90, 100, 200, 300, 400, 500, 600, 700, 800, 900 or about 1000 cubic millimeters in volume.

A preferred stem cell collection composition, useful in placental stem cells by perfusion or physical/enzymatic disruption of placental tissue, comprises one or more tissue-disruptive enzyme(s), e.g., collagenase, dispase, hyaluronidase, LIBERASE (Boehringer Mannheim Corp., Indianapolis, Ind.), papain, deoxyribonucleases, serine proteases, such as trypsin, chymotrypsin, or elastase, or the like. Any combination of tissue digestion enzymes can be used. Typical concentrations for tissue digestion enzymes include, e.g., 50-200 U/mL for collagenase I and collagenase IV, 1-10 U/mL for dispase, and 10-100 U/mL for elastase. Proteases can be used in combination, that is, two or more proteases in the same digestion reaction, or can be used sequentially in order to liberate placental stem cells. For example, in one embodiment, a placenta, or part thereof, is digested first with an appropriate amount of collagenase I at 2 mg/ml for 30 minutes, followed by digestion with trypsin, 0.25%, for 10 minutes, at 37°C Serine proteases are preferably used consecutively following use of other enzymes.

In another embodiment, the tissue can further be disrupted by the addition of a chelator, e.g., ethylene glycol bis(2-aminoethyl ether)-N,N,N'N'-tetraacetic acid (EGTA) or ethylenediaminetetraacetic acid (EDTA) to the stem cell collection composition comprising the stem cells, or to a solution in which the tissue is disrupted and/or digested prior to isolation of the stem cells with the stem cell collection composition.

Where an entire placenta, or portion of a placenta comprising both fetal and maternal cells (for example, where the portion of the placenta comprises the chorion or cotyledons), the placental stem cells collected will comprise a mix of placental stem cells derived from both fetal and maternal sources. Where a portion of the placenta that comprises no, or a negligible number of, maternal cells (for example, amnion), the placental stem cells collected will comprise almost exclusively fetal placental stem cells.

Specific methods of isolating and characterizing placental stem cells are described, e.g., in U.S. Patent Nos. 7,045,148; 7,468,276; 8,057,788 and 8,202,703, the disclosures of which are hereby incorporated by reference in their entireties.

### 6. EXAMPLES

### 6.1. Example 1 Isolation of ECM from Placenta

### 6.1.1. Isolation of ECM

This example illustrates isolation of collagen from placentas.

Frozen placentas are obtained according to the methods described herein. The placentas are thawed by wrapping in a Nalgene tray with water for 1-4 hrs. They are then removed from plastic wrap and placed in water for further thawing. Thawed placentas are placed on the stainless steel tray of a meat grinder. The umbilical cord fragment is cut from each placenta, and each placenta is sliced into about 4 strips at approximately 23°C. The strips are ground with the meat grinder at approximately 23°C.

Osmotic Shock: The resulting ground placental tissue is added to a Nalgene tank with 0.5 M NaCl (5 liters/placenta) and mixed using a motorized mixer at 75-100 rpm (24 hrs at 4-6°C). After 24 hrs, the mixer is stopped, allowing tissue to settle to the bottom of the mixer at approximately 23°C. Tissue and fluid are pumped out using a peristaltic pump with #36 TYGON™ tubing and filtered through a #40 sieve at approximately 23°C., and isolated tissue is placed back into the mixing tank. Fresh 0.5 M NaCl (5 L/placenta) is added to the mixture and mixed for 24 hrs at 4-6°C (motorized mixer, 75-100 rpm). After 24 hrs, the tissue is isolated using the method described above. Tissue is washed with water (5-L/placenta) and mixed for 24 hrs at 4-6°C (motorized mixer, 75-100 rpm). After 24 hrs, the tissue is isolated using the method described above. The tissue is further washed again with 0.5 M NaCl, and then water as above.

Freeze-drying: The resulting tissue is shelled in 200-400 g amounts in a freeze-dryer vessel and frozen at -70°C for 1-2 hrs. The frozen sample is freeze-dried for 24-48 hrs in a freeze-drier and then removed. The freeze-dried sample is mixed to a smooth powder in a blender and then transferred to a clean mixing tank.

Detergent treatment: A 1% deoxycholic acid solution (IL/placenta) is added to the mixing tank with the blended, freeze-dried sample. The sample and 1% deoxycholic acid solution are mixed for 24 hrs at 4-6°C (motorized mixer, 75-100 rpm). After 24 hrs, the mixer is stopped, and tissue is isolated with a #40 sieve as described above. The detergent treatment is repeated for 24 hrs at 4-6°C (motorized mixer, 75-100 rpm). After 24 hrs, the mixer is stopped, and tissue is isolated with a #40 sieve as described above.

Water wash: Tissue is washed with water (5 L/placenta) and mixed for 24 hrs at 4-6°C (motorized mixer, 75-100 rpm). After 24 hrs, the tissue is isolated using the method described above. The tissue is again washed with water (5 L/placenta) and mixed for 24 hrs at 4-6°C (motorized mixer, 100-150 rpm). After 24 hrs, the tissue is isolated using the method described above. The tissue is again washed with water (5 L/placenta) and mixed for 24 hrs at 4-6°C (motorized mixer, 150 rpm). After 24 hrs, the tissue is isolated using the method described above. Optionally, tissue is washed with water (5 L/placenta) a fourth time and mixed for 24 hrs at 4-6°C (motorized mixer, 150 rpm). After 24 hrs, the tissue is isolated using the method described above.

Freeze-drying: The resulting tissue is added to a blender in 200 g amounts. 200 mL deionized water is added to the sample, and the sample is mixed to a smooth paste with the blender. Blended samples are pooled and rinsed with water (1 L/placenta). Tissue in 200-400 g amounts is added to a freeze-dryer vessel. Samples are shelled and frozen at -70°C. Shelled tissue is freeze dried for 24-48 hrs.

Sterile basic treatment: Freeze-dried tissue is pooled. Sodium hydroxide solution (0.5 M, IL) is added to an autoclaved, sterile flask. Low endotoxin water (IL) is added to the pooled, freeze-dried samples. The samples and sodium hydroxide solution are mixed on a shaker at 250 rpm for 4 hrs at approximately 23°C.

Sterile water wash: The sample is recovered by filtration through a sterile #70 filter and rinsed with 1 L endotoxin free water. Endotoxin free water (1 L) is added, and sample is mixed on a shaker at 250 rpm for 18-24 hrs at approximately 23°C. This wash step is repeated another two times. If the pH after three washes is greater than 9, the sample is washed again with endotoxin-free water and mixed on a shaker at about 250 RPM for about 18-24 hours. If the pH is less than or equal to 9, the sample is ready for formulation.

The yield for the above protocol can be 10 g/placenta or more.

The resulting sample can be freeze-dried for storage. For use, the sample can be suspended in phosphate-buffered saline at 300-1000 mg/mL in a blender for use as a paste in, for example, a syringe. The sample can also be molded in phosphate buffered saline at 500-1000 mg/mL and shaped for use as for example, sheets, tubes, plugs, or the like.

### 6.1.2. Preparation of Telopeptide Collagen-Containing ECM Samples

7.5 g of telopeptide collagen-containing ECM was prepared according to the osmotic shock, freeze-drying, detergent treatment, water wash, freeze-drying, basic treatment, water wash and freeze-drying steps of Example 1. 11.8 g of telopeptide collagen-containing ECM was prepared according to the osmotic shock, freeze-drying, detergent treatment, water wash, basic treatment, water wash and freeze-drying steps of Example 1. 12.0 g of telopeptide collagen-containing ECM was prepared according to the osmotic shock, freeze-drying, detergent treatment, water wash, basic treatment, water wash and freeze-drying steps of Example 1. 11.8 g of telopeptide collagen-containing ECM was prepared according to the osmotic shock, detergent treatment, water wash, basic treatment, water wash and freeze-drying steps of Example 1.

### 6.1.3. Analysis of ECM

### Biochemical Analysis:

A collagen sample was prepared according to Examples 1 and 2. Biochemical analysis by standard techniques showed the ECM to be, by dry weight, 80.40% collagen, 1.00% water and less than 0.01% fibronectin, laminin and glycosoaminoglycans. Elastin content was not determined. Amino acid analysis of samples prepared according to Examples 1 and 2 showed 34-35% glycine, about 11% hydroxyproline and 10-11% proline. Immunoanalysis of ECM samples prepared according to Examples 1 and 2 showed 74-92% type I collagen, 4-6% type III collagen and 2-15% type IV collagen.

### 6.1.4. Alternate Methods of Making ECM, and Culture of Stem Cells on the ECM

This Example demonstrates alternate methods of making the collagen composition of the invention, and provides an analysis of the composition of the materials made by those methods.

### Materials and Methods:

Isolation of Extracellular Matrix (ECM): The ECM was isolated as follows. Briefly, a frozen human placenta was thawed in 0.5M sodium chloride, ground in a meat grinder and washed repeatedly in 0.5M sodium chloride and water in a incubator shaker at 23°C, followed by a detergent such as 1% SDS or 0.5% deoxycholic acid. Blood-free placental tissue was treated with 0.1-0.5N sodium hydroxide for times varying between 3 hours and 24 hours to solubilize the cotyledonous tissue, following by rinsing with phosphate-buffered saline (PBS) to neutralize the pH. The material produced as such was a stable paste and was stored at 4°C.

Biochemical Analysis: To determine the biochemical composition of the isolated ECM, a 1 gram sample was freeze-dried and dry weight determined. The ECM was solubilized by either dissolving in 100 mM HCl at 70°C or by pepsin treatment (1 mg/gm) of the ECM in 10 mM HCl at 23°C for 18 hrs. The tissue dissolved in 100 mM HCl was used to determine content of fibronectin, laminin, GAGs and elastin. The pepsin-solubilized tissue was used to determine collagen content.

Fibronectin and laminin concentrations were determined using a sandwich ELISA. Elastin and glycosaminoglycan (GAG) content were determined using a dye based assay. For Determination of collagen I content was performed using a sandwich ELISA (Chondrex). Collagen III and IV content were determined using in-house ELISAs using primary antibodies for Type II and Type IV collagen and HRP-conjugated secondary antibodies.

Preparation of ECM Constructs: To Prepare Sheets of the ECM material, a layer of hydrated ECM paste was sandwiched between two medical grade TYVEK™ sheets. This construct was loaded into a gel drier and vacuum was applied overnight at 23°C until the ECM film was dry. Sheets were cut to an appropriate size for cell culture studies. To prepare 3D structures of the ECM, the ECM paste was filled into various molds and freeze-dried. To study the stability of the ECM sheets and 3D molds in media or water. The constructs were incubated at 37°C up to 1 week, in water, saline or cell culture media.

Cell culture: Placental stem cells were subcultured in 60% low-glucose Dulbecco's Minimal Essential Medium (DMEM) (Invitrogen, Carlsbad, Calif.), 40% MCDB-201 (Sigma, St. Louis, Mo.), 2% fetal bovine serum (Hyclone, Logan, Utah), IX insulin-transferrin-selenium supplement (Invitrogen), 0.02% linoleic acid/bovine serum albumin (Sigma), 10 ng/mL epidermal growth factor (Sigma), 10 ng/mL platelet-derived growth factor (R&D Systems, Minneapolis, Minn.), 0.05M dexamethasone (Sigma), 0.1 mM ascorbic acid 2-phosphate (Sigma), and 100 U penicillin/1000 U streptomycin (Invitrogen). Placental stem cells (30,000 per well) were seeded onto ECM films that had been positioned into 24 multi-well cluster plates. Placental stem cells were also seeded at equivalent density on Labtek chamber slides (Nalgene Nunc International, Rochester, N.Y.) pre-coated with collagen (Inamed, Fremont, Calif.). Cells were incubated at 37°C for 3 and 48 hours and processed for immunofluorescence microscopy.

Immunofluorescence microscopy: After 3 or 48 hr incubation with ECM films, placental stem cell-ECM constructs were fixed with 3.7% formaldehyde for 10 minutes and permeabilized with 0.5% Triton-X 100 for 20 minutes. Placental stem cells were incubated with AlexaFluor 488-conjugated phalloidin to visualize F-actin. For fibronectin staining, samples incubated with a rabbit anti-human fibronectin antibody (Sigma) in blocking buffer (3% bovine serum albumin/1X phosphate-buffered saline) for 1 hour, washed with phosphate-buffered saline, and further incubated with the AlexaFluor 594-conjugated anti-rabbit antibody in blocking buffer for 30 minutes. Samples were again washed with phosphate-buffered saline, mounted on slides, and observed with a fluorescent microscope.

Cytokine secretion analysis: Media samples (100 µL) were removed from cell cultures ECM sheets containing placental stem cells, as well as from tissue culture treated plates containing placental stem cell, at 0, 3, 24 and 48 hrs of culture. Samples were diluted into 1 mL PBS and analyzed for the presence of cytokines. Concentration of each cytokine was calculated from a standard plot of known concentrations of cytokines.

### Results:

Isolation of ECM: The dry weight of a typical placenta is about 30 g, corresponding to a wet weight of about 300 g per placenta. As shown in FIG. 1, the osmotic shock step and detergent washing step can be used to remove a considerable amount of non-extracellular matrix tissue, with a final residual weight of about 10 g. The use of a combination of solubilization using NaOH and detergent results in a further decrease in the residual weight to about 6 g. It was found that the time of exposure to NaOH, and the concentration of NaOH, affected the total mass of ECM isolated from the placenta. Variations of detergent and NaOH wash steps were used to generate 5 variations of the final ECM material, designated ECM-1 through ECM-5. Typically, a single placenta yielded between about 6 g to about 10 g of ECM material.

Biochemical Composition of ECM: Biochemical analysis of the 5 variations of the ECMs showed that they were composed primarily of collagens; Type I being the major collagen (about 74% to about 90% of total collagen), and Type III (about 4% to about 6% of total collagen) and Type IV (about 2% to about 15% of total collagen) being minor components. The other major extracellular matrix protein found in the placental ECM was elastin. As shown in Table 1, elastin represented about 3-5% of the total dry weight of ECM-1 to ECM-4. However, ECM-5, which was generated without the use of NaOH, contained approximately 12% elastin. While glycosaminoglycans were identified in ECM material made by all five methods, % dry weight appeared to be unaffected by the use of NaOH in the isolation methods. The presence of the important adhesion proteins fibronectin and laminin, conversely, was dramatically sensitive to the use of NaOH. Fibronectin and laminin did not survive the NaOH treatment, and could not be found in ECMs 1 through 4. However, ECM-5, which was isolated without the use of NaOH, has a composition that is richer in the adhesion proteins (Table 1).

**TABLE-US-00002 TABLE 1 Extracellular matrix components present in placental collagen compositions made by different methods.**

| | Fibronectin | Laminin | GAGs | Elastin |
|---|---|---|---|---|
| ECM-1 | 0.0% | 0.0% | 0.59% | 3.5% |
| ECM-2 | 0.0% | 0.0% | 0.38% | 4.4% |
| ECM-3 | 0.0% | 0.0% | 0.34% | 3.2% |
| ECM-4 | 0.0% | 0.0% | 0.28% | 4.7% |
| ECM-5 | 0.6% | 0.16% | 0.4% | 12.0% |

Cell Binding Studies: Three hours after seeding, similar levels of attachment of placental stem cells were observed on all of ECMs 1 through 5. The levels of stem cell binding to ECMs were slightly less than that observed on purified collagen. Immunostaining for fibronectin at this time revealed abundant intracellular staining, with no detectable extracellular fibronectin. By 48 hours of culture, placental stem cells were observed to increase in number and to adopt similar well-spread morphologies on purified collagen, ECM-2, and ECM-4. In contrast, placental stem cells cultured on ECM-1 did not thrive. Not only were fewer cells observed, but their morphologies were rounded and not well-spread. Placental stem cells on ECM-5 appeared more elongated and polarized than placental stem cells on other ECMs or on collagen.

Determination of cell attachment on ECM-3 was somewhat compromised due to the heterogeneity of the surface of the material upon drying. Because it was difficult to image along one plane of focus, it initially appeared that very few cells attached; however observation in different planes of focus revealed some cell attachment on ECM-3.

Immunostaining for fibronectin at the 48 hr time point revealed an extensive network of extracellular fibronectin matrix fibrils on ECM-1 through ECM-4. These fibronectin matrix fibrils were assembled by placental stem cells, as controls in which placental stem cells were not cultured on ECM did not show evidence of fibronectin fibrils. In contrast to ECM-1 through ECM-4, ECM-5 and purified collagen did not support fibronectin matrix assembly by placental stem cells; no extracellular fibrillar fibronectin was detected on these surfaces.

Cytokine array studies: We investigated the secretion of key cytokines/chemokines from the placental stem cells as a consequence of binding and proliferation on the ECM. Cytokine secretion on ECM was compared to that from placental stem cells incubated on tissue culture treated cell culture plates. A standard 25-multiplex cytokine array, which includes several interleukins and cytokines (Biosource), was used. The cytokines included IL-1β, IL-IRa, IL-2R, IL-4, IL-5, IL-6, IL-7, IL-8, IL-10, IL-12p40/p70, IL-13, IL-15, IL-17, TNF-α, IFN-α, IFN-γ, GM-CSF, MIP-1α, MIP-1β, IP-10, MIG, Eotaxin, Rantes, and MCP-1. Of the 25 cytokines studied, increased secretion of 3 cytokines, IL-6, IL-8 and MCP-1 were observed when placental stem cells were cultured on the ECM sheets, over and above secretion by placental stem cells cultured on tissue culture treated plates. FIGS. 2A-2C show a time-dependent increase in cytokine secretion (IL-6, IL-8 & MCP-1) by placental stem cells on the five ECM constructs. All data was normalized for 1000 cells bound/cm². ECM-5 was anomalous in that there was no apparent increase in MCP-1 secretion, suggesting a change in cellular physiology of the placental stem cells when cultured on this extra-cellular matrix. As previously shown, ECM-5 did not support the expression of fibronectin, quite unlike ECM-1 through-4. It is interesting to note that ECM-5 was the only matrix generated without the use of NaOH and had a biochemical composition that maintained the 2 key cell adhesion proteins fibronectin and laminin.

### 6.2. Example 2: Generation of Double-Dried ECM

This example illustrates generation of double-dried ECM from placentas.

### 6.2.1. Initial preparation and washing

Placentas were obtained from a normal, full-term delivery and quarantined frozen until the completion of all donor screening tests. Upon being released for processing, the placenta was thawed at room temperature (22°C) for about 24 hrs. The placenta was removed from the container, placed on a sterile tray followed by removal of the amnion, chorionic skirt and umbilical cord. The placenta was cleaned to remove excess blood and blood clots and then cut into approximately 2 X 2 cm segments. The cut placental material was transferred into containers with 1M sterile sodium chloride (1.5L total volume per container). The suspended segments were then homogenized for about 120 seconds using an Omni Mixer homogenizer.

The homogenized tissue was transferred into processing bags and additional 1M sodium chloride sterile solution was added into the bags to make a total volume of 9.2L per single placenta (one batch). The tissue was washed 3 times with a 1 M NaCl sterile solution as follows: the bag with suspended tissue was agitated on a shaker for 3-5 minutes, and the tissue was allowed to settle. Blood and debris were separated from the tissue by removing 6.2L of supernatant via gravity drainage. The washed tissue was allowed to mix in 1M NaCl within the processing bags on an orbital shaker overnight (18-26 hours) at room temperature (22°C). At the end of the overnight period, the tissue was washed 4 times with 6.2 L of sterile water in the manner described above. The washed tissue was allowed to mix in water within the processing bags on an orbital shaker overnight (18-26 hours) at room temperature (22°C).

At the end of the second overnight period, the tissue was washed once with 6.2 L of sterile water in the manner described above. 6.2 L of sterile 3% sodium deoxycholate solution was then added into the bag to make a 2% final concentration of sodium deoxycholate. The tissue was allowed to mix in the sodium deoxycholate solution within the processing bags on an orbital shaker for approximately 72 hrs at room temperature (22°C). At the end of the 72 hr sodium deoxycholate treatment, the tissue was washed five times with 6.2 L of sterile water in the manner described above. After the last fill with sterile water, the pH inside the bag was adjusted to approximately pH 12 with NaOH, and the tissue was allowed to mix in water at this pH on an orbital shaker for about 30 minutes at room temperature (22°C). At the end of the high pH exposure, pH within the bags was adjusted to between pH 5.0 and pH 7.5. Supernatant (6.2L) was removed from the bags and the resulting extracellular matrix (ECM) suspension (approximately 3.0L) was collected into multiple sterile centrifugation bottles. The ECM was then pelleted by centrifugation, followed by decantation of the supernatant. The ECM was washed once with sterile water within the same bottles, centrifuged and the supernatant was again decanted, leaving a washed ECM paste.

### 6.2.2. Drying steps

The ECM paste obtained in Section 6.1.1 was re-suspended in sterile buffer and then transferred into molds. Molds with the ECM suspension were loaded into a controlled rate freezer, and the ECM was frozen at -80°C. Frozen ECM was then stored at -80°C for up to 60 days.

Molds with frozen ECM were loaded into a lyophilizer, where they were freeze-dried using established parameters for up to 72 hours to produce dehydrated ECM (DHCM).

The lyophilized DHCM product (in this example, a 3.5 X 3.5 inch piece) was removed from the molds, packaged, and labeled. Packaged, lyophilized DHCM was optionally stored at room temperature for up to 60 days in unsterilized form (packaged, sterilized DHCM is expected to last up to 5 years).

Non-sterile or sterilized DHCM 3.5"x3.5" wafers were subsequently removed from the pouches and positioned on a sterile Tyvek sheet on a heated vacuum drier platform. The DHCM was completely re-hydrated with buffer, covered with a protective layer of sterile Tyvek, and vacuum-heat-dried at temperatures between 40°C and 80°C for 4-72 hrs.

Upon completion of the drying cycle, DHCM 3.5"x3.5" sheets were removed from the dryer, packaged and labeled.

DHCM 3.5"x3.5" sheets were optionally sterilized by radiation.

All publications and patent applications cited in this specification are herein incorporated by reference. Although the foregoing invention has been described in some detail by way of illustration and example for purposes of clarity of understanding, it will be readily apparent to those of ordinary skill in the art in light of the teachings herein that certain changes and modifications may be made thereto without departing from the spirit or scope of the appended claims.

The following numbered clauses set out preferred features and preferred combinations of features of the present invention:
1. A method of culturing cells, comprising contacting said cells with a plurality of microcarriers under conditions in which said cells can proliferate, wherein said microcarriers comprise extracellular matrix (ECM), and wherein said cells are adherent to said ECM.
2. The method of clause 1, wherein the microcarriers consist essentially of ECM.
3. The method of clause 1, wherein the microcarriers consist of ECM.
4. The method of clause 1, wherein the microcarriers comprise a second composition in addition to said ECM.
5. The method of clause 4, wherein said second composition is one or more of glass, dextran, DEAE-dextran, polystyrene, acrylamide, or collagen.
6. The method of clause 4 or clause 5, wherein said ECM forms an exterior of said microcarrier, and said second composition forms an interior of said microcarrier.
7. The method of any of clauses 4-6, wherein said ECM covers substantially the entire surface of said microcarrier.
8. The method of any of clauses 1-7 wherein the microcarrier is substantially in the shape of a sphere.
9. The method of clause 8, wherein said microcarriers average between 10 micrometers to 1000 micrometers in diameter.
10. The method of clause 8, wherein said microcarriers average between 50 and 200 micrometers in diameter.
11. The method of clause 8, wherein said microcarriers average between 100 and 250 micrometers in diameter.
12. The method of clause 8, wherein said microcarriers average between 150 and 300 micrometers in diameter.
13. The method of clause 8, wherein said microcarriers average between 200 and 350 micrometers in diameter.
14. The method of clause 8, wherein said microcarriers average between 250 and 400 micrometers in diameter.
15. The method of any of clauses 1-14, wherein said culturing is performed in a bioreactor.
16. The method of clause 15, wherein said bioreactor is a batch reactor, a fed batch reactor, or a continuous stirred-tank reactor.
17. The method of any of clauses 1-16, wherein said cells are insect cells.
18. The method of any of clauses 1-16, wherein said cells are mammalian cells.
19. The method of clause 18, wherein said cells are adult cells.
20. The method of clause 19, wherein said cells are fibroblasts.
21. The method of clause 18, wherein said cells are stem cells or progenitor cells.
22. The method of clause 21, wherein said stem cells or progenitor cells are mesenchymal stem cells or mesenchymal-like stem cells.
23. The method of clause 22, wherein said mesenchymal stem cells or mesenchymal-like stem cells are from bone marrow, peripheral blood, umbilical cord blood, placental blood, placental tissue, umbilical cord tissue, Wharton's jelly, amniotic fluid, amnion, chorion, endometrium, adipose tissue, dental pulp, or dermis.
24. The method of any of clauses 1-23, wherein said ECM is base-treated, detergent-treated ECM.
25. The method of any of clauses 1-23, wherein said ECM is detergent-treated ECM that has not been contacted with a base.
26. The method of any of clauses 1-23, wherein said ECM comprises collagen, and comprises between 3% and 5% elastin by dry weight, and less than 0.01% laminin or less than 0.01% fibronectin by dry weight.
27. The method of clause 26, wherein said ECM comprises collagen, and comprises between 3% and 5% elastin by dry weight, less than 0.01% laminin and less than 0.01% fibronectin by dry weight.
28. The method of any of clauses 1-23, wherein said ECM comprises at least 80% collagen by dry weight and at least 10% elastin by dry weight.
29. The method of any of clauses 1-23, wherein said ECM is prepared in part by a method comprising the steps, in order, of: (a) macerating placental tissue; (b) suspending the tissue in an osmotic shock solution; (c) suspending the tissue in a solution that comprises a detergent; (d) suspending the tissue in a basic solution; (e) rinsing said tissue with water; and (f) drying said tissue to form said ECM.
30. The method of any of clauses 1-23, wherein said ECM is prepared in part by a method comprising the steps, in order, of: (a) macerating placental tissue; (b) suspending the tissue in an osmotic shock solution; (c) suspending the tissue in a solution that comprises detergent; (e) rinsing said tissue with water; and (f) drying said tissue to form said ECM.
31. The method of clause 29 or clause 30, wherein said drying comprises the steps, in order, of: (a) lyophilizing the ECM; (b) rehydrating the ECM; and (c) drying the ECM at a temperature of 40°C to 70°C to produce said ECM composition, wherein said method does not comprise modification of the ECM with a chemical or protease.
32. The method of clause 32, wherein said drying the ECM is performed at between 50°C to 70°C.
33. The method of any of clauses 24-31, wherein said detergent is deoxycholic acid.
34. The method of any of clauses 24-31, wherein said base is ammonium hydroxide, potassium hydroxide, or sodium hydroxide.
35. The method of any of clauses 24-31, wherein said base is used at a concentration of less than 0.5M.
36. The method of any of clauses 1-35, wherein said ECM is non-human ECM.
37. The method of any of clauses 1-35, wherein said ECM is human ECM.
38. The method of clause 37, wherein said ECM is from human placenta.

## Claims

1. A method of culturing cells, comprising contacting said cells with a plurality of microcarriers under conditions in which said cells can proliferate, wherein said microcarriers comprise extracellular matrix (ECM), and wherein said cells are adherent to said ECM.

2. The method of claim 1, wherein the microcarriers;
(i) consist essentially of ECM;
(ii) consist of ECM; or
(iii) comprise a second composition in addition to said ECM.

3. The method of claim 2 item (iii), wherein said second composition is one or more of glass, dextran, DEAE-dextran, polystyrene, acrylamide, or collagen.

4. The method of claim 3, wherein;
(i) said ECM forms an exterior of said microcarrier, and said second composition forms an interior of said microcarrier; and/or
(ii) said ECM covers substantially the entire surface of said microcarrier.

5. The method of any of claims 1-4, wherein the microcarrier is substantially in the shape of a sphere.

6. The method of claim 5, wherein said microcarriers average between 10 micrometers to 1000 micrometers in diameter; between 50 and 200 micrometers in diameter; between 100 and 250 micrometers in diameter; between 150 and 300 micrometers in diameter; between 200 and 350 micrometers in diameter; or between 250 and 400 micrometers in diameter.

7. The method of any of claims 1-6, wherein said culturing is performed in a bioreactor; preferably wherein said bioreactor is a batch reactor, a fed batch reactor, or a continuous stirred-tank reactor.

8. The method of any of claims 1-7, wherein said cells are insect cells or mammalian cells.

9. The method of claim 8, wherein said mammalian cells are:
(i) adult cells, preferably fibroblasts; or
(ii) stem cells or progenitor cells, preferably mesenchymal stem cells or mesenchymal-like stem cells.

10. The method of claim 9 item (ii), wherein said mesenchymal stem cells or mesenchymal-like stem cells are from bone marrow, peripheral blood, umbilical cord blood, placental blood, placental tissue, umbilical cord tissue, Wharton's jelly, amniotic fluid, amnion, chorion, endometrium, adipose tissue, dental pulp, or dermis.

11. The method of any of claims 1-10, wherein said ECM;
(i) is base-treated, detergent-treated ECM;
(ii) is detergent-treated ECM that has not been contacted with a base;
(iii) comprises collagen, and comprises between 3% and 5% elastin by dry weight, and less than 0.01% laminin or less than 0.01 % fibronectin by dry weight;
(iv) comprises collagen, and comprises between 3%> and 5%> elastin by dry weight, less than 0.01% laminin and less than 0.01% fibronectin by dry weight;
(v) comprises at least 80% collagen by dry weight and at least 10% elastin by dry weight;
(vi) is prepared in part by a method comprising the steps, in order, of:
(a) macerating placental tissue;
(b) suspending the tissue in an osmotic shock solution;
(c) suspending the tissue in a solution that comprises a detergent;
(d) suspending the tissue in a basic solution;
(e) rinsing said tissue with water; and
(f) drying said tissue to form said ECM; or
(vii) is prepared in part by a method comprising the steps, in order, of:
(a) macerating placental tissue;
(b) suspending the tissue in an osmotic shock solution;
(c) suspending the tissue in a solution that comprises detergent; (e) rinsing said tissue with water; and
(f) drying said tissue to form said ECM.

12. The method of claim 11 item (vi) or item (vii), wherein said drying comprises the steps, in order, of:
(a) lyophilizing the ECM;
(b) rehydrating the ECM; and
(c) drying the ECM at a temperature of 40°C to 70°C to produce said ECM composition, wherein said method does not comprise modification of the ECM with a chemical or protease; preferably wherein said drying the ECM is performed at between 50°C to 70°C.

13. The method of any of claims 11 or 12, wherein said detergent is deoxycholic acid.

14. The method of any of claims 11 or 12, wherein said base is ammonium hydroxide, potassium hydroxide, or sodium hydroxide; and/or wherein said base is used at a concentration of less than 0.5M.

15. The method of any of claims 1-14, wherein said ECM is non-human ECM or human ECM, preferably wherein said ECM is from human placenta.
